# EUROPEAN PATENT SPECIFICATION

(11) **EP 3 268 391 B1**
(45) Date of publication and mention of the grant of the patent: **11.08.2021**
(21) Application number: 16719896.9
(22) Date of filing: 08.03.2016
(51) Int. Cl.: C07K 16/00, A61K 38/17, A61K 45/06, C07K 16/28, C07K 16/40, C07K 14/47, A61K 39/00

(54) **METHODS OF REDUCING SERUM LEVELS OF FC-CONTAINING AGENTS USING FCRN ANTAGONSITS**
VERFAHREN ZUR REDUZIERUNG DER SERUMSPIEGEL VON FC-HALTIGEN MITTELN UNTER VERWENDUNG VON FCRN-ANTAGONISTEN
PROCÉDÉS DE RÉDUCTION DE NIVEAUX DE SÉRUM D'AGENTS CONTENANT FC À L'AIDE D'ANTAGONISTES FCRN

(30) Priority: 09.03.2015 US 201562130076 P
(43) Date of publication of application: 17.01.2018
(73) Proprietor: argenx BVBA, 9052 Zwijnaarde (BE)
(72) Inventor: ONGENAE, Nicolas, G.h., 4300 Waremme (BE); DREIER, Torsten, B-9830 Sint Martens Latern (BE); ULRICHTS, Peter, B-9070 Destelbergen (BE); DE HAARD, Johannes, Oudelande NA 4436 (NL); BLANCHETOT, Christophe, 9070 Destelbergen (BE)
(74) Representative: Boult Wade Tennant LLP
(86) International application number: PCT/IB2016/000398
(87) International publication number: WO 2016/142782

(56) References cited:
- WO-A1-2013/074598
- WO-A1-2015/100299
- WO-A2-2006/130834
- WO-A2-2013/063186
- D. A. PATEL ET AL: "Neonatal Fc Receptor Blockade by Fc Engineering Ameliorates Arthritis in a Murine Model", THE JOURNAL OF IMMUNOLOGY, vol. 187, no. 2, 15 July 2011 (2011-07-15) , pages 1015-1022, XP055292443, US ISSN: 0022-1767, DOI: 10.4049/jimmunol.1003780 cited in the application
- DILIP K. CHALLA ET AL: "Autoantibody depletion ameliorates disease in murine experimental autoimmune encephalomyelitis", MABS, vol. 5, no. 5, 1 September 2013 (2013-09-01), pages 655-659, XP055292468, US ISSN: 1942-0862, DOI: 10.4161/mabs.25439
- Argen-X: "arGEN-X N.V.", , 20 June 2014 (2014-06-20), XP055292474, Retrieved from the Internet: URL:https://www.afm.nl/registers/emissies_ documents/14414.pdf [retrieved on 2016-08-01]
- Anonymous: "arGEN-X advances ARGX-113 into preclinical development for autoimmune disorders - Argenx", , 24 April 2014 (2014-04-24), XP055292450, Retrieved from the Internet: URL:http://www.argen-x.com/en-GB/news-inte rnal/argen-x-advances-argx-113-into-precli nical-development-for-autoimmune-disorders /60/ [retrieved on 2016-08-01]
- Anonymous: "arGEN-X announces positive preclinical results for ARGX-113 | Euronext", , 19 August 2014 (2014-08-19), XP055292448, Retrieved from the Internet: URL:https://www.euronext.com/nl/node/50665 2 [retrieved on 2016-08-01]
- CARLOS VACCARO ET AL: "Engineering the Fc region of immunoglobulin G to modulate in vivo antibody levels", NATURE BIOTECHNOLOGY, vol. 23, no. 10, 1 October 2005 (2005-10-01), pages 1283-1288, XP055049342, ISSN: 1087-0156, DOI: 10.1038/nbt1143
- Quinlin Hanson: "The role of the Immunoglobulin G1 Fc N-glycan in Fc[gamma]RIIIa affinity", , 1 January 2014 (2014-01-01), XP055292649, ISBN: 978-1-321-59176-7 Retrieved from the Internet: URL:http://lib.dr.iastate.edu/cgi/viewcont ent.cgi?article=5142&context=etd [retrieved on 2016-08-03]
- Sandra Grau ET AL: "IgG core a-fucosylation and its impact on FcgRIIIa binding", , 21 September 2011 (2011-09-21), XP055292648, Retrieved from the Internet: URL:http://www.cisbio.com/sites/default/fi les/ressources/cisbio-pr-igg-core-a-fucosy lation-and-its-impact-on-fc-gamma-riiia-bi nding.pdf [retrieved on 2016-08-01]
- YUTAKA KANDA ET AL: "Comparison of biological activity among nonfucosylated therapeutic IgG1 antibodies with three different N-linked Fc oligosaccharides: the high-mannose, hybrid, and complex types", GLYCOBIOLOGY, OXFORD UNIVERSITY PRESS, US, vol. 17, no. 1, 1 January 2007 (2007-01-01), pages 104-118, XP002696785, ISSN: 0959-6658, DOI: 10.1093/GLYCOB/CWL057 [retrieved on 2006-09-29]

## Description

### BACKGROUND

Immunoglobulin gamma (IgG) antibodies play a key role in the pathology of many disorders, such as autoimmune diseases, inflammatory diseases, and disorders in which the pathology is characterized by over-expression of IgG antibodies (e.g., hypergammaglobulinemia) (see e.g. Junghans, Immunologic Research 16 (1):29 (1997)).

The half-life of IgG in the serum is prolonged relative to the serum half-life of other plasma proteins (Roopenian et al., J. Immunology 170:3528 (2003); Junghans and Anderson, Proc. Natl. Acad. Sci. USA 93:5512 (1996)). This long half-life is due, in part, to the binding of the Fc region of IgG to the Fc receptor, FcRn. Although FcRn was originally characterized as a neonatal transport receptor for maternal IgG, it also functions in adults to protect IgG from degradation. FcRn binds to pinocytosed IgG and protects the IgG from transport to degradative lysosomes by recycling it back to the extracellular compartment. This recycling is facilitated by the pH dependent binding of IgG to FcRn, where the IgG/FcRn interaction is stronger at acidic endosomal pH than at extracellular physiological pH.

When the serum concentration of IgG reaches a level that exceeds available FcRn molecules, unbound IgG is not protected from degradative mechanisms and will consequently have a reduced serum half-life. Thus, inhibition of IgG binding to FcRn reduces the serum half-life of IgG by preventing IgG endosomal recycling of IgG. Accordingly, agents that antagonize the binding of IgG to FcRn may be useful for regulating, treating or preventing antibody-mediated disorders, such as autoimmune diseases, inflammatory diseases, etc. One example of a method of antagonizing IgG Fc binding to FcRn involves the generation of blocking antibodies to FcRn (see e.g. WO2002/43658). Peptides have also been identified that bind to and antagonize FcRn function (see e.g. US6,212,022 and US8,101,186). In addition, full-length IgG antibodies comprising variant Fc receptors with enhanced FcRn binding and decreased pH dependence have also been identified that antagonize FcRn binding to IgG (see e.g. US8,163,881). However, there is a need in the art for improved methods for the treatment of antibody-mediated disorders.

### SUMMARY

The present disclosure relates to novel methods of reducing the serum levels of Fc-containing agents (e.g., antibodies and immunoadhesins) in a subject. These methods generally comprise administering to the subject an effective amount of an isolated FcRn-antagonist that binds specifically to FcRn with increased affinity and reduced pH dependence relative to the native Fc region. The described methods are particularly useful for treating antibody-mediated disorders (e.g. autoimmune diseases).

Accordingly, the present invention provides an isolated FcRn-antagonist consisting of a variant Fc region for use in a method of treating an autoimmune disease in a subject, the method comprising administering to the subject an effective amount of the isolated FcRn-antagonist consisting of the variant Fc region wherein the amino acid sequence of the Fc domains of the variant Fc region consists of the amino acid sequence set forth in SEQ ID NO:1, 2, or 3, wherein the FcRn-antagonist is administered intravenously, wherein the FcRn-antagonist is administered to the subject in a dose of between 2 and 200 mg/kg, and wherein the FcRn-antagonist is administered to the subject at a frequency of once every 7 days.

In accordance with the claims, the FcRn-antagonist is administered to the subject at a frequency of once every 7 days.

In accordance with the claims, the FcRn-antagonist is administered to the subject in a dose of between 2 and 200 mg/kg.

In accordance with the claims, the FcRn-antagonist is administered intravenously.

The FcRn-antagonist does not comprise an antibody variable region. The FcRn-antagonist does not comprise a CH1 domain. The variant Fc region is an IgG1 Fc region. In certain embodiments, the amino acid sequence of the Fc domains of the variant Fc region consists of the amino acid sequence set forth in SEQ ID NO:1. In accordance with the claims, the FcRn-antagonist consists of a variant Fc region, wherein the amino acid sequence of the Fc domains of the variant Fc region consists of the amino acid sequence set forth in SEQ ID NO:1, 2, or 3.

In certain embodiments, the variant Fc region has an increased affinity for an Fc gamma receptor relative to the affinity of a wild-type IgG1 Fc region for the Fc gamma receptor. In certain embodiments, the variant Fc region has increased affinity for CD16a. In certain embodiments, the Fc domains of the variant Fc region do not comprise an N-linked glycan at EU position 297.

In certain embodiments, the variant Fc region is linked to a half-life extender. In certain embodiments, the half-life extender is polyethylene glycol or human serum albumin.

The subject has an antibody-mediated disease or disorder, as defined in the claims, wherein administration of the FcRn-antagonist to the subject ameliorates the disease or disorder. In certain embodiments, the disease or disorder is treatable using intravenous immunoglobulin (IVIG), plasmapheresis and/or immunoadsorption. In accordance with the claims, the antibody-mediated disease or disorder is an autoimmune disease. In certain embodiments, the autoimmune disease is selected from the group consisting of allogenic islet graft rejection, alopecia areata, ankylosing spondylitis, antiphospholipid syndrome, autoimmune Addison's disease, Alzheimer's disease, antineutrophil cytoplasmic autoantibodies (ANCA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune disfunction syndrome, chronic inflammatory demyelinating polyneuropathy (CIDP), Churg-Strauss syndrome, cicatrical pemphigoid, CREST syndrome, cold agglutinin disease, Crohn's disease, dermatomyositis, dilated cardiomyopathy, discoid lupus, epidermolysis bullosa acquisita, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic membranous neuropathy, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen plantus, lichen sclerosus, lupus erthematosis, Meniere's disease, mixed connective tissue disease, mucous membrane pemphigoid, multiple sclerosis, type 1 diabetes mellitus, Multifocal motor neuropathy (MMN), myasthenia gravis, paraneoplastic bullous pemphigoid, pemphigoid gestationis, pemphigus vulgaris, pemphigus foliaceus, pernicious anemia, polyarteritis nodosa, polychrondritis, polyglandular syndromes, polymyalgia rheumatica, polymyositis and dermatomyositis, primary agammaglobinulinemia, primary biliary cirrhosis, psoriasis, psoriatic arthritis, relapsing polychondritis, Reynauld's phenomenon, Reiter's syndrome, rheumatoid arthritis, sarcoidosis, scleroderma, Sjorgen's syndrome, solid organ transplant rejection, stiff-man syndrome, systemic lupus erythematosus, takayasu arteritis, toxic epidermal necrolysis (TEN), Stevens Johnson syndrome (SJS), temporal arteristis/giant cell arteritis, thrombotic thrombocytopenia purpura, ulcerative colitis, uveitis, dermatitis herpetiformis vasculitis, anti-neutrophil cytoplasmic antibody-associated vasculitides, vitiligo, and Wegner's granulomatosis.

In certain embodiments, the autoimmune disease is an autoimmune channelopathy. In certain embodiments, the channelopathy is selected from the group consisting of autoimmune limbic encephalitis, epilepsy, neuromyelitis optica, Lambert-Eaton myasthenic syndrome, myasthenia gravis, anti- N-Methyl-D-aspartate (NMDA) receptor encephalitis, anti-α-Amino-3-hydroxy-5-methyl-4-isoxazolepropionic acid (AMPA) receptor encephalitis, Morvan syndrome, neuromyotonia, pediatric autoimmune neuropychiatric disorders associated with streptococcal infection (PANDAS), and Glycine receptor antibody-associated disorder. In certain embodiments, the antibody-mediated disorder is hyperglobulinemia.

In certain embodiments, the FcRn-antagonist is administered to the subject simultaneously or sequentially with an additional therapeutic agent. In certain embodiments, the additional therapeutic agent is an anti-inflammatory agent. In certain embodiments, the additional therapeutic agent is a leucocyte depleting agent. In certain embodiments, the leucocyte depleting agent is a B-cell depleting agent. In certain embodiments, the B-cell depleting agent is an antibody. In certain embodiments, the B-cell depleting agent is an antibody that specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86. In certain embodiments, the additional therapeutic agent is rituximab, daclizumab, basiliximab, muronomab-CD3, infliximab, adalimumab, omalizumab, efalizumab, natalizumab, tocilizumab, eculizumab, golimumab, canakinumab, ustekinumab, belimumab, or a combination thereof.

In certain embodiments, the subject is a human or cynomolgus monkey.

### BRIEF DESCRIPTION OF THE DRAWINGS

FIG.1 depicts the results of experiments to determine the effect of Fc-Abdeg and HEL-Abdeg on the serum levels of a tracer antibody (FR70-hIgG1) in cynomolgus monkey.
FIG.2 depicts the results of experiments to determine the effect of Fc-Abdeg and HEL-Abdeg on total IgG serum levels in cynomolgus monkey.
FIG.3 depicts the results of experiments to determine the effect of Fc-Abdeg and HEL-Abdeg on albumin levels in cynomolgus monkey.
FIG.4 depicts the results of experiments to determine the effect of Fc-Abdeg and IVIG on the serum levels of a tracer antibody (FR70-hIgG1) in cynomolgus monkey.
FIG.5 depicts the results of ELISA assays comparing the affinity of Fc-Abdeg, Fc-Abdeg-POT and Fc-Abdeg-S239D/I332E for human CD16a.
FIG.6 depicts the results of ELISA assays comparing the affinity of Fc-Abdeg, Fc-Abdeg-POT and Fc-Abdeg-S239D/I332E for murine CD16-2.
FIG.7 depicts the results of experiments to determine the effect of Fc-Abdeg, Abdeg-POT and Fc-AbdegS239D/I332E on anti-CD20-induced ADCC-signal using the Promega's Raji-based ADCC reporter bioassay.
FIG.9 depicts the results of experiments to determine the effect of Fc-Abdeg and Abdeg-POT on anti-CD70-induced lysis of CD70+ U266 cells *in vitro.*

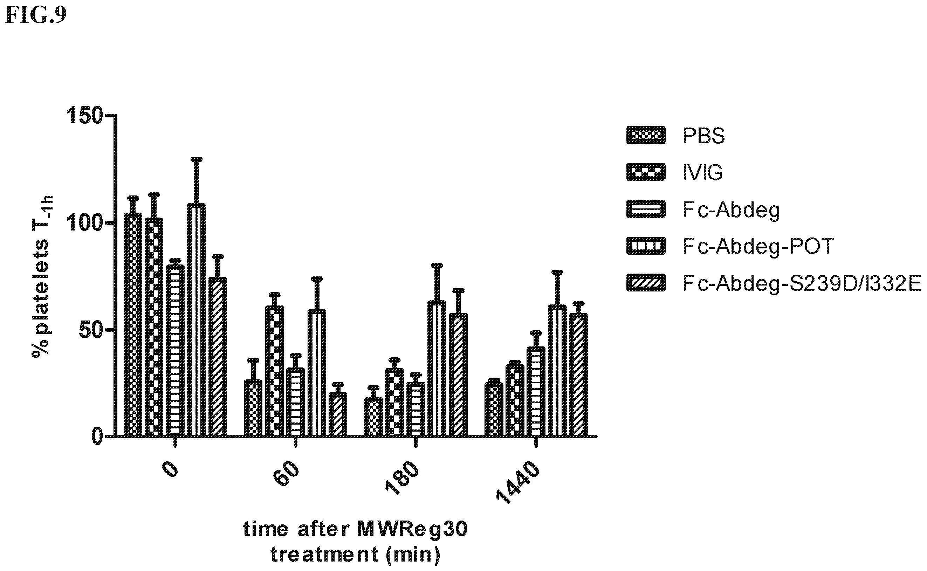

FIG.9 depicts the results of experiments to determine the effect of Fc-Abdeg, Fc-Abdeg-POT, Fc-Abdeg-S239D/I332E and IVIG on platelet levels in an acute murine model for immune thrombocytopenia.

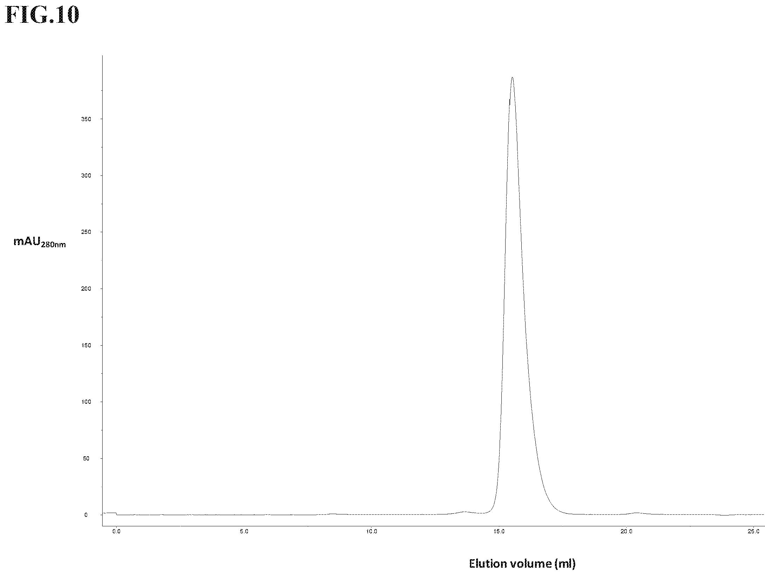

FIG.10 depicts the result of an exemplary gel filtration purification of Fc-Abdeg.

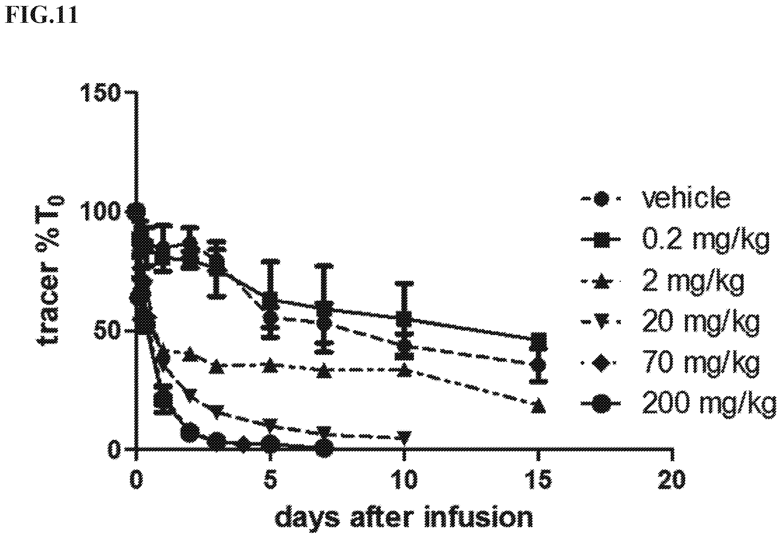

FIG.11 depicts the results of a dose-escalation study measuring the effect of various single doses of Fc-Abdeg on the serum levels of a tracer antibody (FR70-hIgG1) in cynomolgus monkey.

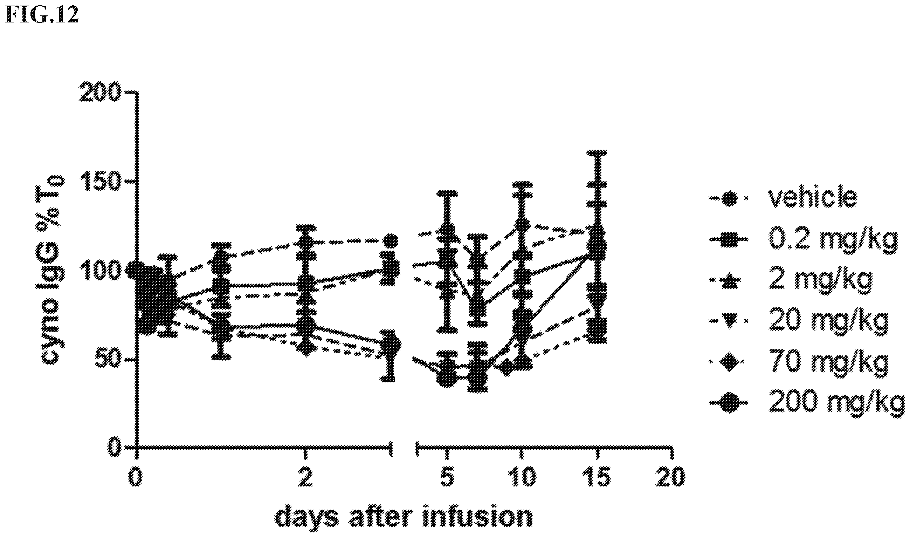

FIG.12 depicts the results of a dose-escalation study measuring the effect of various single doses of Fc-Abdeg on the serum levels of a tracer antibody (FR70-hIgG1) in cynomolgus monkey.

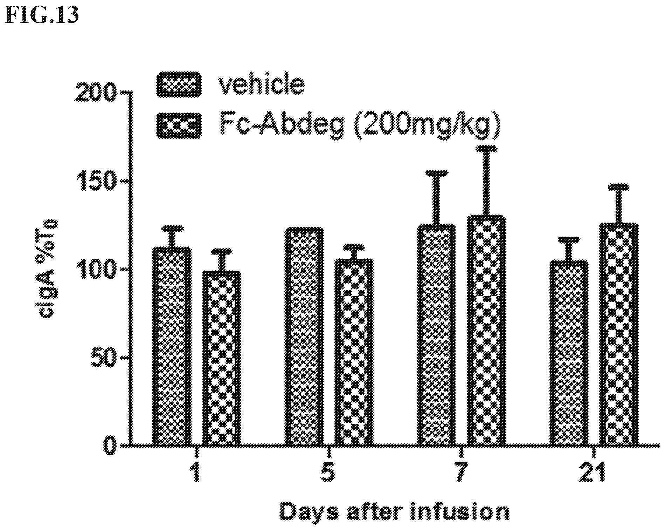

FIG.13 depicts the results of experiments to determine the effect of 200 mg/kg Fc-Abdeg on cIgA levels in cynomolgus monkey.

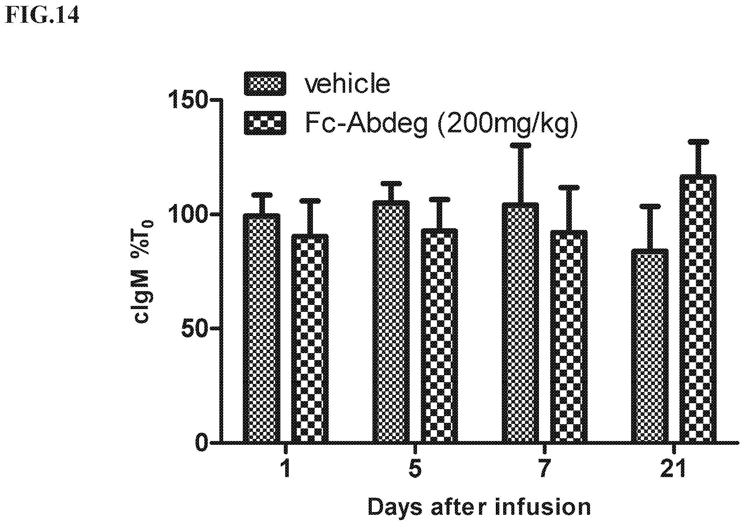

FIG. 14 depicts the results of experiments to determine the effect of 200 mg/kg Fc-Abdeg on cIgM levels in cynomolgus monkey.

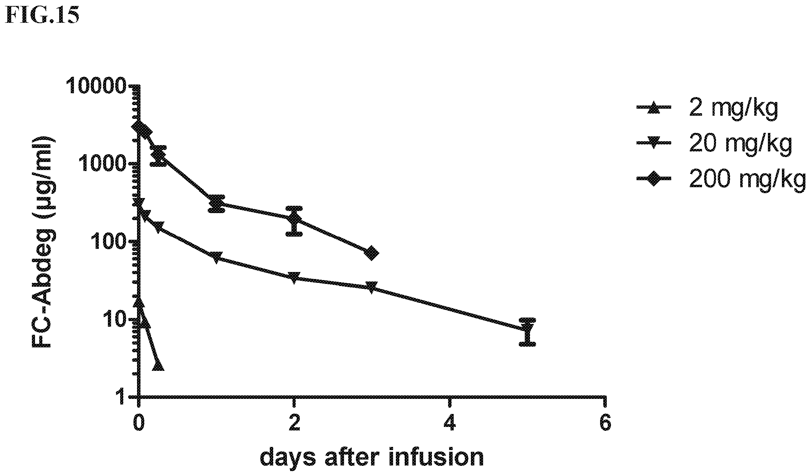

FIG. 15 depicts the pharmacokinetic profile of various single doses of Fc-Abdeg in cynomolgus monkey.

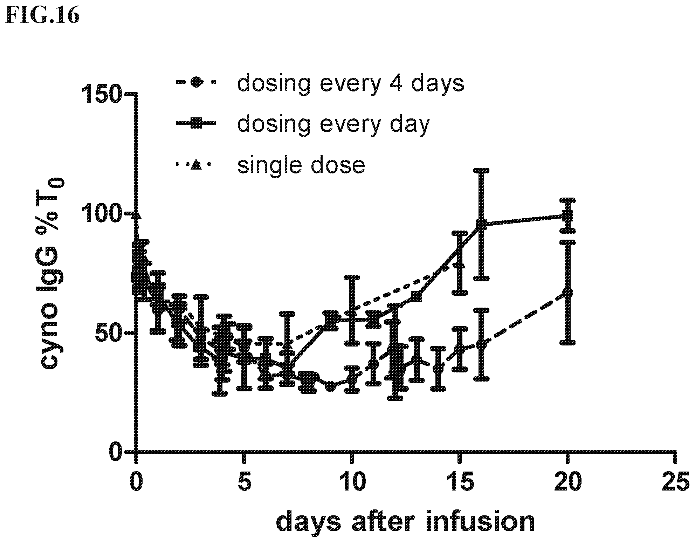

FIG. 16 depicts the results of experiments to determine the effect of repetitive multiple doses of 20 mg/kg Fc-Abdeg on cIgG levels in cynomolgus monkey.

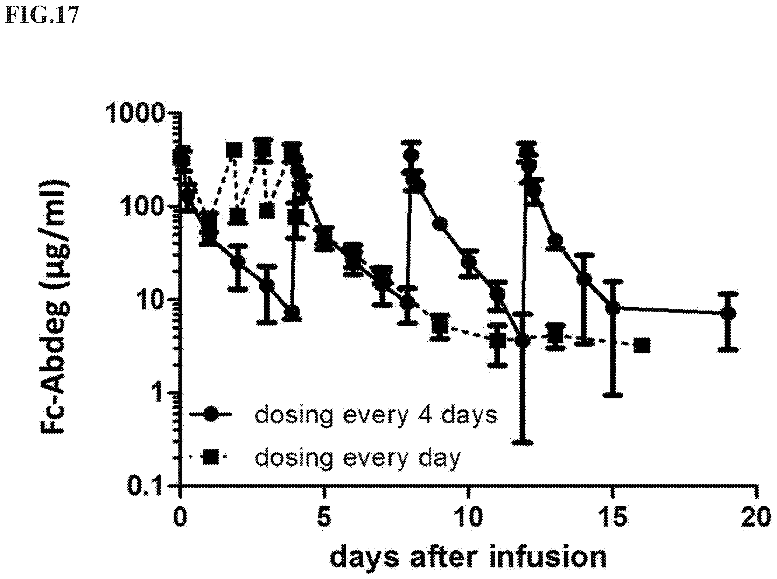

FIG. 17 depicts the pharmacokinetic profile of Fc-Abdeg in cynomolgus monkey given repetitive multiple doses of 20 mg/kg Fc-Abdeg.

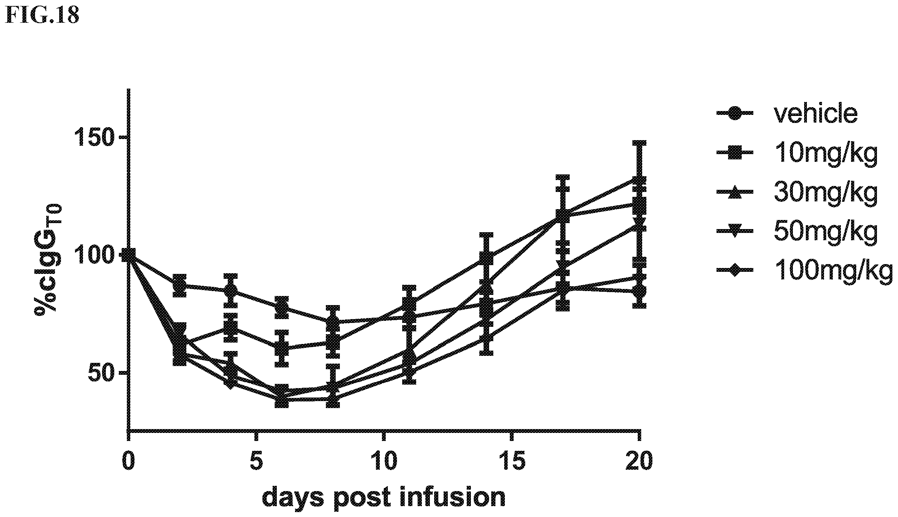

FIG.18 depicts the results of a dose-escalation study measuring the effect of various single doses of Fc-Abdeg on the serum levels of endogenous IgG in cynomolgus monkey.

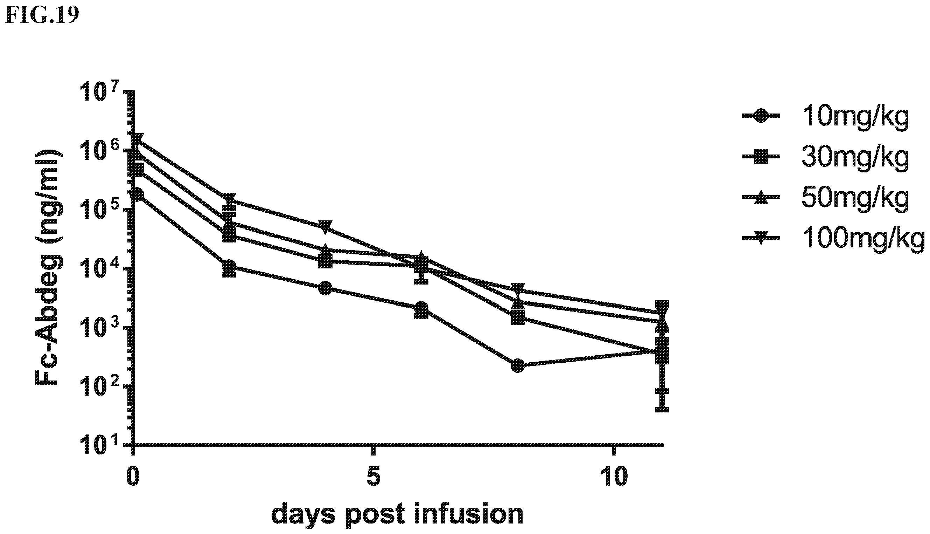

FIG. 19 depicts the pharmacokinetic profile of various single doses of Fc-Abdeg in cynomolgus monkey.

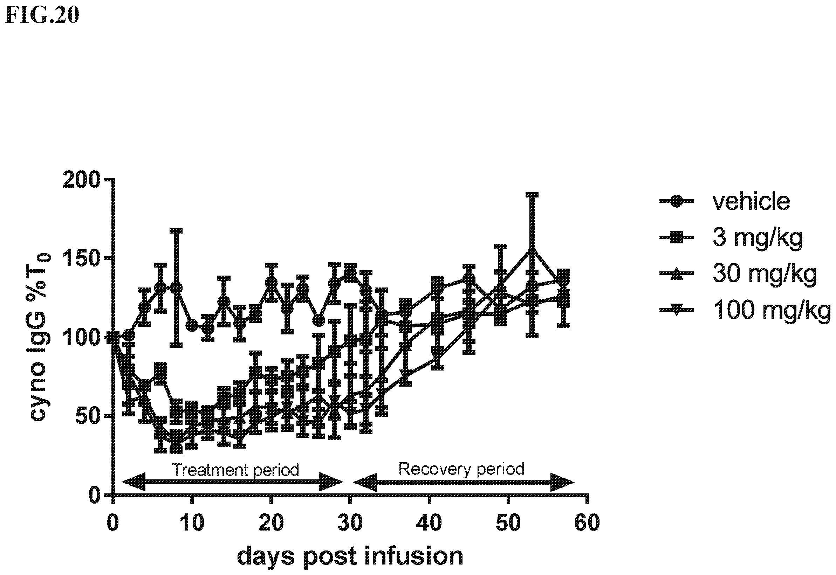

FIG.20 depicts the results of a repetitive dosing study measuring the effect of various repetitive doses of Fc-Abdeg on the serum levels of endogenous IgG in cynomolgus monkey.

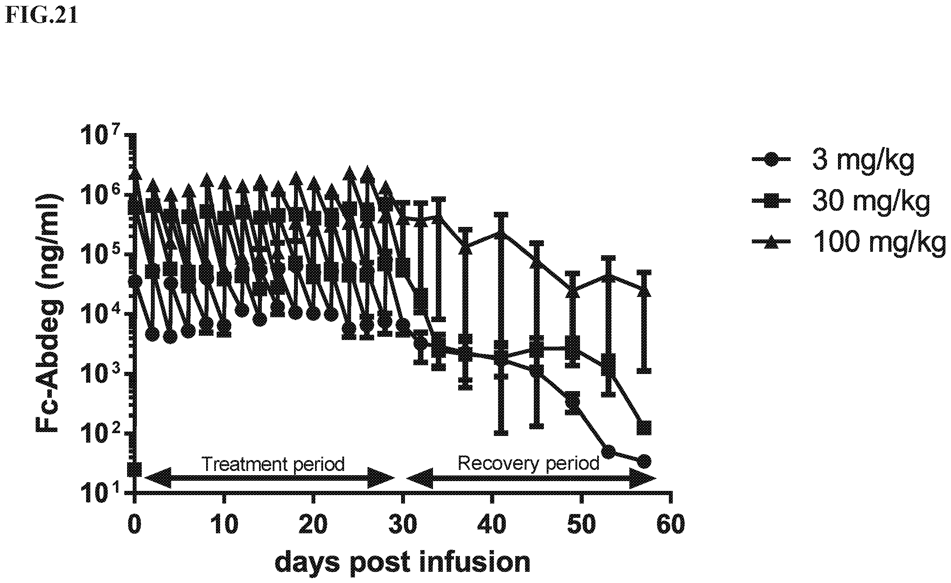

FIG.21 depicts the pharmacokinetic profile of various repetitive doses of Fc-Abdeg in cynomolgus monkey.

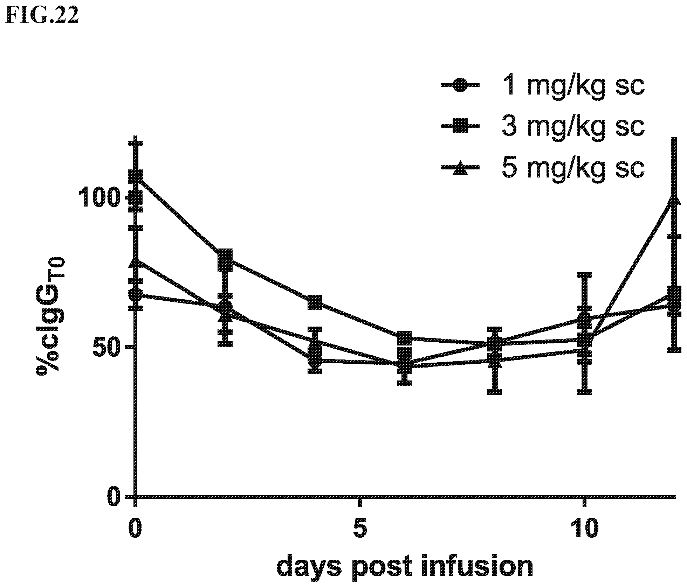

FIG.22 depicts the results of a continuous dosing study measuring the effect of various doses of Fc-Abdeg on the serum levels of endogenous IgG in cynomolgus monkey.

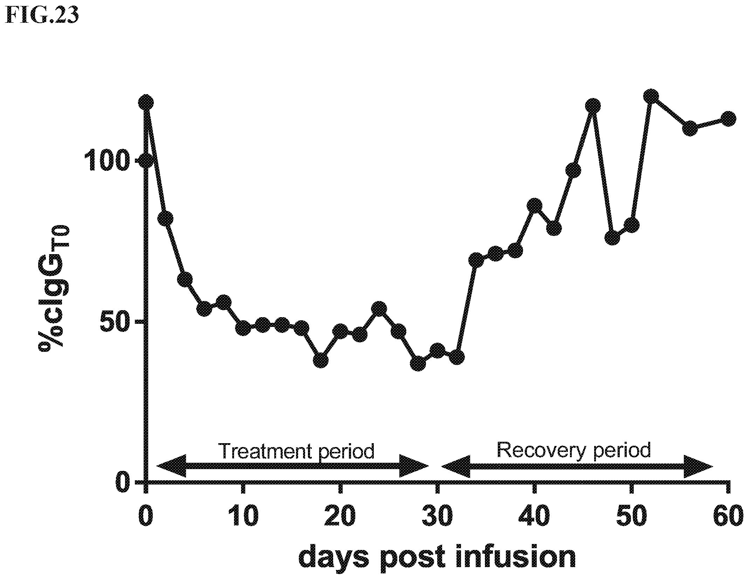

FIG.23 depicts the results of a continuous dosing study measuring the effect of an intravenous loading dose of 20 mg/kg Fc-Abdeg followed 24 hours later by daily subcutaneous administration of Fc-Abdeg at 3 mg/kg for 28 days and a subsequent treatment-free period of 32 days in a cynomolgus monkey subject.

### DETAILED DESCRIPTION

The present disclosure relates to novel methods of reducing the serum levels of Fc-containing agents (e.g., antibodies and immunoadhesins) in a subject. These methods generally comprise administering to the subject an effective amount of an isolated FcRn-antagonist that binds specifically to FcRn with increased affinity and reduced pH dependence relative to the native Fc region. The described methods are particularly useful for treating antibody-mediated disorders (e.g. autoimmune diseases).

### I. Definitions

Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition. Further, unless otherwise required by context, singular terms shall include pluralities and plural terms shall include the singular. Generally, nomenclature used in connection with, and techniques of, cell and tissue culture, molecular biology, immunology, microbiology, genetics and protein and nucleic acid chemistry and hybridization described herein are those well known and commonly used in the art.

In order that the present invention may be more readily understood, certain terms are first defined.

As used herein the term "FcRn-antagonist" refers to any agent comprising an Fc region (e.g., a variant Fc region disclosed herein) that binds specifically to FcRn through the Fc region and inhibits the binding of immunoglobulin to FcRn, with the proviso that the agent is not a full length IgG antibody.

As used herein, the term "Fc region" refers to the portion of a native immunoglobulin formed by the Fc domains of its two heavy chains. A native Fc region is homodimeric.

As used herein, the term "variant Fc region" refers to an Fc region with one or more alterations relative to a native Fc region. Alteration can include amino acid substitutions, additions and/or deletions, linkage of additional moieties, and/or alteration to the native glycans. The term encompasses heterodimeric Fc regions where each of the constituent Fc domains is different. Examples of such heterodimeric Fc regions include, without limitation, Fc regions made using the "knobs and holes" technology as described in, for example, US 8216805. The term also encompasses single chain Fc regions where the constituent Fc domains are linked together by a linker moiety, as described in, for example, US20090252729A1 and US20110081345A1.

As used herein, the term "Fc domain" refers to the portion of a single immunoglobulin heavy chain beginning in the hinge region just upstream of the papain cleavage site and ending at the C-terminus of the antibody. Accordingly, a complete Fc domain comprises at least a portion of a hinge (e.g., upper, middle, and/or lower hinge region) domain, a CH2 domain, and a CH3 domain.

As used herein the term "FcRn binding fragment" refers to a portion of an Fc region that is sufficient to confer FcRn binding.

As used herein, the term "EU position" refers to the amino acid position in the EU numbering convention for the Fc region described in Edelman, G.M. et al., Proc. Natl. Acad. USA, 63, 78-85 (1969) and Kabat et al, in "Sequences of Proteins of Immunological Interest", U.S. Dept. Health and Human Services, 5th edition, 1991.

As used herein, the term "CH1 domain" refers to the first (most amino terminal) constant region domain of an immunoglobulin heavy chain that extends from about EU positions 118-215. The CH1 domain is adjacent to the VH domain and amino terminal to the hinge region of an immunoglobulin heavy chain molecule, and does not form a part of the Fc region of an immunoglobulin heavy chain.

As used herein, the term "hinge region" refers to the portion of a heavy chain molecule that joins the CH1 domain to the CH2 domain. This hinge region comprises approximately 25 residues and is flexible, thus allowing the two N-terminal antigen binding regions to move independently. Hinge regions can be subdivided into three distinct domains: upper, middle, and lower hinge domains (Roux et al. J. Immunol. 161: 4083 (1998)). The FcRn-antagonists described herein can include all or a portion of a hinge region.

As used herein, the term "CH2 domain" refers to the portion of a heavy chain immunoglobulin molecule that extends from about EU positions 231-340.

As used herein, the term "CH3 domain" includes the portion of a heavy chain immunoglobulin molecule that extends approximately 110 residues from N-terminus of the CH2 domain, e.g., from about position 341-446 (EU numbering system).

As used herein, the term "FcRn" refers to a neonatal Fc receptor. Exemplary FcRn molecules include human FcRn encoded by the FCGRT gene as set forth in RefSeq NM_004107.

As used herein, the term "CD16" refers to FcγRIII Fc receptors that are required for Antibody-Dependent Cell-mediated Cytotoxicity (ADCC). Exemplary CD16 molecules include human CD16a as set forth in RefSeq NM_000569.

As used herein, the term "free cysteine" refers to native or engineered cysteine amino acid residue that exists in a substantially reduced form in a mature FcRn-antagonist.

As used herein, the term "antibody" refers to immunoglobulin molecules comprising four polypeptide chains, two heavy (H) chains and two light (L) chains interconnected by disulfide bonds, as well as multimers thereof (e.g., IgM). Each heavy chain comprises a heavy chain variable region (abbreviated VH) and a heavy chain constant region. The heavy chain constant region comprises three domains, CH1, CH2 and CH3. Each light chain comprises a light chain variable region (abbreviated VL) and a light chain constant region. The light chain constant region comprises one domain (CL). The VH and VL regions can be further subdivided into regions of hypervariability, termed complementarity determining regions (CDRs), interspersed with regions that are more conserved, termed framework regions (FR).

As used herein the term "N-linked glycan" refers to the N-linked glycan attached to the nitrogen (N) in the side chain of asparagine in the sequon (i.e., Asn-X-Ser or Asn-X-Thr sequence, where X is any amino acid except proline) present in the CH2 domain of an Fc region. Such N-Glycans are fully described in, for example, Drickamer K, Taylor ME (2006). Introduction to Glycobiology, 2nd ed.

As used herein the term "afucosylated" refers to an N-linked glycan which lacks a core fucose molecule as described in US8067232.

As used herein the term "bisecting GlcNac" refers to an N-linked glycan having an N-acetylglucosamine (GlcNAc) molecule linked to a core mannose molecule, as described in US8021856.

As used herein, the term "antibody-mediated disorder" refers to any disease or disorder caused or exacerbated by the presence of an antibody in a subject.

As used herein, the term "Fc-containing agent" is any molecule that comprises an Fc region.

As used herein, the term "leucocyte depleting agent" refers to an agent that reduces the number of leucocytes in a subject upon administration.

As used herein, the term "B-cell depleting agent" refers to an agent that reduces the number of B-cells in a subject upon administration.

As used herein, the term "T-cell depleting agent" refers to an agent that reduces the number of T-cells in a subject upon administration.

As used herein, the term "autoimmune channelopathy" refers to a diseases caused by autoantibodies against an ion channel subunit or a molecule that regulates the channel.

As used herein, the term "treat," "treating," and "treatment" refer to therapeutic or preventative measures described herein. The methods of "treatment" employ administration of an antibody or antigen binding fragment thereof described herein, for example, to a subject having an IL-6-associated disease or disorder (e.g. inflammation and cancer) or predisposed to having such a disease or disorder, in order to prevent, cure, delay, reduce the severity of, or ameliorate one or more symptoms of the disease or disorder or recurring disease or disorder, or in order to prolong the survival of a subject beyond that expected in the absence of such treatment. As used herein, the term "subject" includes any human or non-human animal.

As used herein, the term "immunoadhesin" refers to an antibody-like molecule, which comprises a functional domain of a binding protein (e.g., a receptor, ligand, or cell-adhesion molecule) with an Fc region.

### II. Methods of Reducing Serum Levels of Fc-Containing Agents

Described herein are methods of reducing the serum levels of Fc-containing agents (e.g., antibodies and immunoadhesins) in a subject, the methods comprising administering to the subject an effective amount of an isolated FcRn-antagonist that binds specifically to FcRn with increased affinity and reduced pH dependence relative to the native Fc region (e.g., FcRn-antagonists disclosed herein).

As shown herein, administration of an FcRn-antagonist to the subject in a dose of between about 0.2 and about 200 mg/kg is unexpectedly efficacious. Accordingly, as described herein, the FcRn-antagonist may be administered to the subject in a dose of between about 0.2 and about 200 mg/kg (e.g., between 0.2 and 200 mg/kg). In certain embodiments, the FcRn-antagonist is administered to the subject in a dose of about 0.2, 2, 20, 70, or 200 mg/kg (e.g., 0.2, 2, 20, 70, or 200 mg/kg). In certain embodiments, the FcRn-antagonist is administered to the subject in a dose of about 20 mg/kg (e.g., 20 mg/kg). In accordance with the claims, the FcRn-antagonist is administered to the subject in a dose of between 2 and 200 mg/kg.

As shown herein, a multiple, repeated dosing regime is unexpectedly superior to a single dose. Accordingly, as described herein, the FcRn-antagonist may be administered to the subject at least twice in 20 days. As described herein, the FcRn-antagonist may be administered to the subject at a frequency of once every 1, 2, 3, 4, 5, 6, 7, 8, 9, or 10 days. As described herein, the FcRn-antagonist may be administered to the subject 2, 3, 4, 5, 6, 7, 8, 9, 10, 11, 12, 13, 14, 15, 16, 17, 18, 19, or 20 times in 20 days. In certain examples, the FcRn-antagonist is administered to the subject at a frequency of once every 4 days. In certain examples, the FcRn-antagonist is administered to the subject every 4 days for 13 days (i.e., on days 1, 5, 9, and 13). In certain examples, 20 mg/kg of the FcRn-antagonist is administered to the subject every 4 days for 13 days (i.e., on days 1, 5, 9, and 13). In accordance with the claims, the FcRn-antagonist is administered to the subject at a frequency of once every 7 days

As described herein, the FcRn-antagonist can be administered by any means to the subject. Methods of administration include, but are not limited to, intradermal, intramuscular, intraperitoneal, intravenous, subcutaneous, intranasal, epidural, and oral routes. The composition may be administered, for example by infusion or bolus injection. In accordance with the claims, the FcRn-antagonist is administered intravenously.

The methods described herein can reduce the serum levels of any Fc-containing agent. In certain embodiments, the Fc-containing agent is an antibody or immunoadhesin. In certain examples, the Fc-containing agent is a therapeutic or diagnostic agent. In certain examples, the Fc-containing agent is an imaging agent. In certain embodiments the Fc-containing agent is an antibody-drug conjugate. In certain embodiments, the Fc-containing agent is a pathogenic antibody, e.g., an autoantibody. In certain embodiments, the subject has an antibody-mediated disorder a disease or disorder. In certain embodiments, antibody-mediated disorder a disease or disorder is associated with an autoantibody.

The reduction of serum levels of Fc-containing agents (e.g., antibodies and immunoadhesins) is particularly applicable to the treatment of antibody-mediated disorders (e.g. autoimmune diseases). Accordingly, the present invention relates to methods of treating a subject having an antibody-mediated disorder (e.g. an autoimmune disease), the method comprising administering to the subject an effective amount of an FcRn-antagonist composition disclosed herein.

Any antibody-mediated disorder can be treated using the methods described herein. In certain embodiments, the antibody-mediated disorder is one that is amenable to treatment by IVIG. In accordance with the claims, the antibody-mediated disorder is an autoimmune disease. Non-limiting autoimmune diseases include allogenic islet graft rejection, alopecia areata, ankylosing spondylitis, antiphospholipid syndrome, autoimmune Addison's disease, Alzheimer's disease, antineutrophil cytoplasmic autoantibodies (ANCA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune disfunction syndrome, chronic inflammatory demyelinating polyneuropathy (CIDP), Churg-Strauss syndrome, cicatrical pemphigoid, CREST syndrome, cold agglutinin disease, Crohn's disease, dermatomyositis, discoid lupus, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen plantus, lupus erthematosis, Meniere's disease, mixed connective tissue disease, multiple sclerosis, type 1 diabetes mellitus, Multifocal motor neuropathy (MMN), myasthenia gravis, paraneoplastic bullous pemphigoid, pemphigus vulgaris, pemphigus foliaceus, pernicious anemia, polyarteritis nodosa, polychrondritis, polyglandular syndromes, polymyalgia rheumatica, polymyositis and dermatomyositis, primary agammaglobinulinemia, primary biliary cirrhosis, psoriasis, psoriatic arthritis, Reynauld's phenomenon, Reiter's syndrome, rheumatoid arthritis, sarcoidosis, scleroderma, Sjorgen's syndrome, solid organ transplant rejection, stiff-man syndrome, systemic lupus erythematosus, takayasu arteritis, toxic epidermal necrolysis (TEN), Stevens Johnson syndrome (SJS), temporal arteristis/giant cell arteritis, thrombotic thrombocytopenia purpura, ulcerative colitis, uveitis, dermatitis herpetiformis vasculitis, anti-neutrophil cytoplasmic antibody-associated vasculitides, vitiligo, and Wegner's granulomatosis.

In certain embodiments, the autoimmune disease is an autoimmune channelopathy. Non-limiting channelopathies include neuromyelitis optica, Lambert-Eaton myasthenic syndrome, myasthenia gravis, anti- N-Methyl-D-aspartate (NMDA) receptor encephalitis, anti-α-Amino-3-hydroxy-5-methyl-4-isoxazolepropionic acid (AMPA) receptor encephalitis, Morvan syndrome, and Glycine receptor antibody-associated disorder.

The FcRn-antagonists for use, as disclosed herein, are particularly suited to treating antibody-mediated disorders characterized by an over production of serum immunoglobulin. Accordingly, in certain embodiments, the FcRn-antagonist compositions are used to treat hypergammaglobulinemia.

The FcRn-antagonists for use, as disclosed herein, can also be used in combination with one or more additional therapeutic agents. In certain embodiments, the additional therapeutic agent is an anti-inflammatory agent. Any inflammatory agent can be used in combination with the compositions disclosed herein. In certain embodiments, the therapeutic agent is rituximab, daclizumab, basiliximab, muronomab-cd3, infliximab, adalimumab, omalizumab, efalizumab, natalizumab, tocilizumab, eculizumab, golimumab, canakinumab, ustekinumab, or belimumab. In certain embodiments, the additional therapeutic agent is leucocyte depleting agent (e.g., B-cell or T-cell depleting agent). Any leucocyte depleting agent can be used in combination with the FcRn-antagonists disclosed herein. In certain embodiments, the leucocyte depleting agent is a B-cell depleting agent. In certain embodiments, the leucocyte depleting agent is an antibody against a cell surface marker. Suitable cell surface markers include, without limitation, CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86. The FcRn-antagonist and the additional therapeutic agent(s) can be administered to the subject simultaneously or sequentially, via the same or different route(s) of administration.

The FcRn-antagonists for use, as disclosed herein,are also well suited to rapidly reducing the serum levels of an Fc-containing agent in subject. Such rapid clearance is advantageous in cases where the Fc-containing agent is toxic (e.g., an antibody-drug conjugate or an agent that is immunogenic) because it reduces the exposure of the subject to the drug. Rapid clearance is also advantageous in cases where the Fc-containing agent is an imaging agent that requires a low serum level of the agent to facilitate imaging. Accordingly, in certain embodiments, the FcRn-antagonist compositions are used to reduce the serum levels of an Fc-containing agent (e.g., an imaging agent) in subject that has been administered the Fc-containing agent. The serum levels of any Fc-containing agent (e.g., therapeutic or diagnostic agent) can be reduced using the FcRn-antagonists disclosed herein. Non limiting examples of Fc-containing agents include imaging agents (e.g., labeled antibodies), antibody drug conjugates, or immunogenic agents (e.g., non-human antibodies or immunoadhesins). The FcRn-antagonist can be administered simultaneously with the Fc-containing agent or sequentially (e.g., before or after the Fc-containing agent).

Furthermore, in diseases or conditions requiring administration of a therapeutic agent, the subject will often develop antibodies (e.g., anti-drug antibodies) against the therapeutic agent, which, in turn, prevent the therapeutic agent from being available for its intended therapeutic purpose or cause an adverse reaction in the subject. Accordingly, the FcRn-antagonists for use, as disclosed herein, can also be used to remove antibodies (e.g., anti-drug antibodies) against the therapeutic agent that develop in a subject.

The FcRn-antagonists for use, as disclosed herein, can also be used in combination with a therapeutic protein to enhance the benefit of the therapeutic protein by reducing the levels of IgG; wherein, IgG antibodies are responsible for the decreased bioavailability of a therapeutic protein. In accordance with the claims, the FcRn-antagonist is for use in a method of treating an autoimmune disease. In certain embodiments the FcRn-antagonist is for use in a method of treating a subject having a disorder resulting from an immune response to a clotting factor, the method comprising administering to a subject a therapeutically effective amount of an FcRn-antagonist disclosed herein. Suitable clotting factors include, without limitation, fibrinogen, prothrombin, factor V, factor VII, factor VIII, factor IX, factor X, factor XI, factor XII, factor XIII, or von Willebrand's factor. This method may be used to regulate or treat, or prevent an immune response to a clotting factor in a patient suffering, e.g., from hemophilia A or hemophilia B. In certain embodiments, the method may be used to regulate or treat an immune response to, e.g., therapeutic erythropoietin in a patient suffering from pure red cell aplasia (PRCA).

FcRn is responsible for transporting maternal antibodies across the placenta to the fetus in a pregnant woman. Accordingly, if a pregnant female is administered an Fc-containing agent (e.g., a therapeutic antibody), the agent may come in contact with the fetus as a result of the FcRn-mediated transport across the placenta. To avoid any potential deleterious effect of the Fc-containing agent on fetal development, it would be advantageous to block FcRn function. Accordingly, described herein is a method of preventing placental transfer of an Fc-containing agent (e.g., a therapeutic antibody) to the fetus in a pregnant woman, the method comprising administering to the woman an FcRn-antagonist compositions disclosed herein, either simultaneously or sequentially (prior or post) with the Fc-containing agent.

The methods described herein can also be used to treat inflammatory disorders including, but not limited to, asthma, ulcerative colitis and inflammatory bowel syndrome allergy, including allergic rhinitis/sinusitis, skin allergies (urticaria/hives, angioedema, atopic dermatitis), food allergies, drug allergies, insect allergies, mastocytosis, arthritis, including osteoarthritis, rheumatoid arthritis, and spondyloarthropathies.

Successful implementation of gene therapy for the treatment of a disease or condition may be hampered by the development of antibodies specific to the therapeutic protein encoded by the transgene as well as possibly to the vector used to deliver the transgene. Accordingly, the FcRn-antagonist compositions disclosed herein can be administered in combination with gene therapy to enhance the benefit of the encoded therapeutic protein by reducing the levels of IgG. These methods are particularly useful in situations where IgG antibodies are responsible for the decreased bioavailability of a gene therapy vector or the encoded therapeutic protein. The gene therapy vector may be, e.g., a viral vector such as adenovirus and adeno-associated virus. Diseases that can be treated using gene therapy include, but are not limited to, cystic fibrosis, hemophilia, PRCA, muscular dystrophy, or lysosomal storage diseases, such as, e.g., Gaucher's disease and Fabry's disease.

Any subject can be treated using the methods disclosed herein. In certain embodiments, the subject is a human or a cynomolgus monkey.

### III. FcRn-Antagonists

The methods described herein generally comprise administering to a subject an effective amount of an isolated FcRn-antagonist, wherein the FcRn-antagonist binds specifically to FcRn with increased affinity and reduced pH dependence relative to the native Fc region. In general, these FcRn-antagonists comprise a variant Fc region, or FcRn-binding fragment thereof, that binds specifically to FcRn with increased affinity and reduced pH dependence relative to a native Fc region. The FcRn-antagonists inhibit the binding of Fc-containing agents (e.g., antibodies and immunoadhesins) to FcRn *in vivo,* which results in an increased rate of degradation of the Fc-containing agents and, concomitantly, a reduced serum level of these agents.

As shown herein, an isolated variant Fc region (e.g., a variant Fc region comprising the amino acids Y, T, E, K, F, and Y at EU positions 252, 254, 256, 433, 434, and 436 respectively) is a more efficacious FcRn-antagonist *in vivo* than a full-length antibody comprising the same variant Fc region. Accordingly, the FcRn-antagonists as defined in the claims are not full-length antibodies. The FcRn-antagonist compositions do not comprise an antibody variable domain. The FcRn-antagonist compositions do not comprise an antibody variable domain or a CH1 domain.

Any Fc region can be altered to produce a variant Fc region. In general, an Fc region, or FcRn-binding fragment thereof, is from a human immunoglobulin. It is understood, however, that the Fc region may be derived from an immunoglobulin of any other mammalian species, including for example, a Camelid species, a rodent (e.g. a mouse, rat, rabbit, guinea pig) or non-human primate (e.g. chimpanzee, macaque) species. Moreover, the Fc region or portion thereof may be derived from any immunoglobulin class, including IgM, IgG, IgD, IgA and IgE, and any immunoglobulin isotype, including IgGl, IgG2, IgG3 and IgG4. The Fc region as defined in the claims is an IgG Fc region (e.g., a human IgG region). The Fc region as defined in the claims is an IgG1 Fc region (e.g., a human IgG1 region). In certain examples, the Fc region is a chimeric Fc region comprising portions of several different Fc regions. Suitable examples of chimeric Fc regions are set forth in US20110243966A1. A variety of Fc region gene sequences (e.g. human constant region gene sequences) are available in the form of publicly accessible deposits.

An Fc region can be further truncated or internally deleted to produce a minimal FcRn-binding fragment thereof. The ability of an Fc-region fragment to bind to FcRn can be determined using any art recognized binding assay e.g., ELISA.

To enhance the manufacturability of the FcRn-antagonists disclosed herein, it is preferable that the constituent Fc regions do not do comprise any non-disulphide bonded cysteine residues. Accordingly, in certain embodiments the Fc regions do not comprise a free cysteine residue.

Various Fc variants, or FcRn-binding fragments thereof, that binds specifically to FcRn with increased affinity and reduced pH dependence relative to the native Fc region are described herein. In certain embodiments, the variant Fc region comprises amino acid alterations, substitutions, insertions and/or deletions that confer the desired characteristics. In certain embodiments, the variant Fc region or fragment comprises the amino acids Y, T, E, K, F, and Y at EU positions 252, 254, 256, 433, 434, and 436 respectively. Non-limiting examples of amino acid sequences that can be used in variant Fc regions are set forth in Table 1, herein. In certain embodiments, the amino acid sequence of the Fc domains of the variant Fc region consists of the amino acid sequence set forth in SEQ ID NO:1. In accordance with the claims, the amino acid sequence of the Fc domains of the variant Fc region consists of the amino acid sequence set forth in SEQ ID NO:1, 2, or 3. In accordance with the claims, an FcRn-antagonist consists of a variant Fc region, wherein the amino acid sequence of the Fc domains of the variant Fc region consists of the amino acid sequence set forth in SEQ ID NO:1, 2, or 3.

**Table 1. Amino acid sequences of non-limiting examples of variant Fc regions**

| **SEQ ID NO** | **Amino Acid Sequence** |
|---|---|
| 1 | CPPCPAPELLGGPSVFLFPPKPKDTLYITREPEVTCVVVDVSHEDPEVKFNWYVD GVEVHNAKTKPREEQYNSTYRVVSVLTVLHQDWLNGKEYKCKVSNKALPAPIEKT ISKAKGQPREPQVYTLPPSRDELTKNQVSLTCLVKGFYPSDIAVEWESNGQPENN YKTTPPVLDSDGSFFLYSKLTVDKSRWQQGNVFSCSVMHEALKFHYTQKSLSLSP G |
| 2 | DKTHTCPPCPAPELLGGPSVFLFPPKPKDTLYITREPEVTCVVVDVSHEDPEVKF NWYVDGVEVHNAKTKPREEQYNSTYRVVSVLTVLHQDWLNGKEYKCKVSNKALPA PIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALKFHYTQKS LSLSPGK |
| 3 | DKTHTCPPCPAPELLGGPSVFLFPPKPKDTLYITREPEVTCVVVDVSHEDPEVKF NWYVDGVEVHNAKTKPREEQYNSTYRVVSVLTVLHQDWLNGKEYKCKVSNKALPA PIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALKFHYTQKS LSLSPG |
| Amino acids at EU positions 252, 254, 256, 433, and 434 are underlined | |

In certain embodiments, the variant Fc region has altered (e.g., increased or decreased) binding affinity for an additional Fc receptor. The variant Fc region can have altered (e.g., increased or decreased) binding affinity for one or more of Fcγ receptors e.g., FcγRI (CD64), FcγRIIA (CD32), FcγRIIB (CD32), FcγRIIIA (CD16a), and FcγRIIIB (CD16b). Any art recognized means of altering the affinity for an additional Fc receptor can be employed. In certain embodiments, the amino acid sequence of the variant Fc region is altered.

In certain embodiments, the variant Fc region comprises a non-naturally occurring amino acid residue at one or more positions selected from the group consisting of 234, 235, 236, 239, 240, 241, 243, 244, 245, 247, 252, 254, 256, 262, 263, 264, 265, 266, 267, 269, 296, 297, 298, 299, 313, 325, 326, 327, 328, 329, 330, 332, 333, and 334 as numbered by the EU index as set forth in Kabat. Optionally, the Fc region may comprise a non-naturally occurring amino acid residue at additional and/or alternative positions known to one skilled in the art (see, e.g., U.S. Pat. Nos. 5,624,821; 6,277,375; 6,737,056; PCT Patent Publications WO 01/58957; WO 02/06919; WO 04/016750; WO 04/029207; WO 04/035752 and WO 05/040217).

In certain embodiments, the variant Fc region comprises at least one non-naturally occurring amino acid residue selected from the group consisting of 234D, 234E, 234N, 234Q, 234T, 234H, 234Y, 2341, 234V, 234F, 235A, 235D, 235R, 235W, 235P, 235S, 235N, 235Q, 235T, 235H, 235Y, 2351, 235V, 235F, 236E, 239D, 239E, 239N, 239Q, 239F, 239T, 239H, 239Y, 2401, 240A, 240T, 240M, 241W, 241 L, 241Y, 241E, 241R. 243W, 243L 243Y, 243R, 243Q, 244H, 245A, 247V, 247G, 252Y, 254T, 256E, 2621, 262A, 262T, 262E, 2631, 263A, 263T, 263M, 264L, 2641, 264W, 264T, 264R, 264F, 264M, 264Y, 264E, 265G, 265N, 265Q, 265Y, 265F, 265V, 2651, 265L, 265H, 265T, 2661, 266A, 266T, 266M, 267Q, 267L, 269H, 269Y, 269F, 269R, 296E, 296Q, 296D, 296N, 296S, 296T, 296L, 2961, 296H, 269G, 297S, 297D, 297E, 298H, 2981, 298T, 298F, 2991, 299L, 299A, 299S, 299V, 299H, 299F, 299E, 313F, 325Q, 325L, 3251, 325D, 325E, 325A, 325T, 325V, 325H, 327G, 327W, 327N, 327L, 328S, 328M, 328D, 328E, 328N, 328Q, 328F, 3281, 328V, 328T, 328H, 328A, 329F, 329H, 329Q, 330K, 330G, 330T, 330C, 330L, 330Y, 330V, 3301, 330F, 330R, 330H, 332D, 332S, 332W, 332F, 332E, 332N, 332Q, 332T, 332H, 332Y, and 332A as numbered by the EU index as set forth in Kabat. Optionally, the Fc region may comprise additional and/or alternative non-naturally occurring amino acid residues known to one skilled in the art (see, e.g., U.S. Pat. Nos. 5,624,821; 6,277,375; 6,737,056; PCT Patent Publications WO 01/58957; WO 02/06919; WO 04/016750; WO 04/029207; WO 04/035752 and WO 05/040217).

Other known Fc variants include without limitations those disclosed in Ghetie et al., 1997, Nat. Biotech. 15:637-40; Duncan et al, 1988, Nature 332:563-564; Lund et al., 1991, J. Immunol., 147:2657-2662; Lund et al, 1992, Mol. Immunol., 29:53-59; Alegre et al, 1994, Transplantation 57:1537-1543; Hutchins et al., 1995, Proc Natl. Acad Sci USA, 92:11980-11984; Jefferis et al, 1995, Immunol Lett., 44:111-117; Lund et al., 1995, Faseb J., 9:115-119; Jefferis et al, 1996, Immunol Lett., 54:101-104; Lund et al, 1996, J. Immunol., 157:4963-4969; Armour et al., 1999, Eur J Immunol 29:2613-2624; Idusogie et al, 2000, J. Immunol., 164:4178-4184; Reddy et al, 2000, J. Immunol., 164:1925-1933; Xu et al., 2000, Cell Immunol., 200:16-26; Idusogie et al, 2001, J. Immunol., 166:2571-2575; Shields et al., 2001, J Biol. Chem., 276:6591-6604; Jefferis et al, 2002, Immunol Lett., 82:57-65; Presta et al., 2002, Biochem Soc Trans., 30:487-490); U.S. Pat. Nos. 5,624,821; 5,885,573; 5,677,425; 6,165,745; 6,277,375; 5,869,046; 6,121,022; 5,624,821; 5,648,260; 6,528,624; 6,194,551; 6,737,056; 6,821,505; 6,277,375; U.S. Patent Publication Nos. 2004/0002587 and PCT Publications WO 94/29351; WO 99/58572; WO 00/42072; WO 02/060919; WO 04/029207; WO 04/099249; WO 04/063351.

In certain embodiments, the variant Fc region is a heterodimer, where the constituent Fc domains are different from each other. Methods of producing Fc heterodimers are known in the art (see e.g., US 8216805). In certain embodiments, the variant Fc region is a single chain Fc region, where the constituent Fc domains are linked together by a linker moiety. Methods of producing single chain Fc regions are known in the art (see e.g., US20090252729A1 and US20110081345A1).

It is believed that pathogenic IgG antibodies observed in autoimmune diseases are either the pathogenic triggers for these diseases or contribute to disease progression and mediate disease through the inappropriate activation of cellular Fc receptors. Aggregated autoantibodies and/or autoantibodies complexed with self antigens (immune complexes) bind to activating Fc receptors, causing numerous autoimmune diseases (which occur in part because of immunologically mediated inflammation against self tissues) (see e.g., Clarkson et al., NEJM 314(9), 1236-1239 (2013)); US20040010124A1; US20040047862A1; and US2004/0265321A1). Accordingly, to treat antibody-mediated disorders (e.g. autoimmune diseases), it would be advantageous to both remove the deleterious autoantibodies and to block the interaction of the immune complexes of these antibodies with activating Fc receptors (e.g., Fcγ receptors, such as CD16a).

Accordingly, in certain embodiments, the variant Fc region of the FcRn-antagonist exhibits increased binding to CD16a (e.g., human CD16a). This is particularly advantageous in that it allows the FcRn-antagonist to additionally antagonize the immune complex-induced inflammatory response of autoantibodies being targeted for removal by FcRn inhibition. Any art recognized means of increasing affinity for CD16a (e.g., human CD16a) can be employed. In certain embodiments, the FcRn-antagonist comprises a variant Fc-region comprising an N-linked glycan (e.g., at EU position 297). In this case it is possible to increase the binding affinity of the FcRn-antagonist for CD16a by altering the glycan structure. Alterations of the N-linked glycan of Fc regions are well known in the art. For example, afucosylated N-linked glycans or N-glycans having a bisecting GlcNac structure have been shown to exhibit increased affinity for CD16a. Accordingly, in certain embodiments, the N-linked glycan is afucosylated. Afucosylation can be achieved using any art recognized means. For example, an FcRn-antagonist can be expressed in cells lacking fucosyl transferase, such that fucose is not added to the N-linked glycan at EU position 297 of the variant Fc region (see e.g., US 8,067,232). In certain embodiments, the N-linked glycan has a bisecting GlcNac structure. The bisecting GlcNac structure can be achieved using any art recognized means. For example, an FcRn-antagonist can be expressed in cells expressing betal-4-N-acetylglucosaminyltransferase III (GnTIII) , such that bisecting GlcNac is added to the N-linked glycan at EU position 297 of the variant Fc region (see e.g., US 8021856). Additionally or alternatively, alterations of the N-linked glycan structure can also be achieved by enzymatic means *in vitro.*

In certain examples described herein, the FcRn-antagonist comprises a plurality of FcRn-antagonist molecules, wherein at least 50% (optionally, at least 60, 70, 80, 90, 95, or 99%) of the plurality of FcRn-antagonist molecules comprise a variant Fc region, or FcRn-binding fragment thereof, comprising an afucosylated N-linked glycan at EU position 297.

In certain examples described herein, the FcRn-antagonist comprises a plurality of FcRn-antagonist molecules, wherein at least 50% (optionally, at least 60, 70, 80, 90, 95, or 99%) of the plurality of FcRn-antagonist molecules comprise a variant Fc region or FcRn-binding fragment thereof, comprising an N-linked glycan having a bisecting GlcNac at EU position 297.

In certain embodiments, the variant Fc region does not comprise an N-linked glycan. This can be achieved using any art recognized methods. For example, the Fc variant can be expressed in a cell that is incapable of N-linked glycosylation. Additionally or alternatively, the amino acid sequence of the Fc variant can be altered to prevent or inhibit N-linked glycosylation (e.g., by mutation of the NXT sequon). Alternatively, the Fc variant can be synthesized in an acellular system (e.g., chemically synthesized).

In certain embodiments, FcRn-antagonist molecules may be modified, e.g., by the covalent attachment of a molecule (e.g., a binding or imaging moiety) to the FcRn-antagonist such that covalent attachment does not prevent the FcRn-antagonist from specifically binding to FcRn. For example, but not by way of limitation, the FcRn-antagonist may be modified by glycosylation, acetylation, pegylation, phosphorylation, amidation, derivatization by known protecting blocking groups, proteolytic cleavage, linkage to a cellular ligand or other protein, etc.

In certain embodiments, the FcRn-antagonist comprises a variant Fc region linked to a half-life extender. As used herein, the term "half-life extender" refers to any molecule that, when linked to an FcRn-antagonist disclosed herein, increases the half-life of an FcRn-antagonist. Any half-life extender may be linked (either covalently or non-covalently) to the FcRn-antagonist. In certain embodiments, the half-life extender is polyethylene glycol or human serum albumin. In certain embodiments, the FcRn-antagonist is linked to a binding molecule that specifically binds to a half-life extender present in a subject, such as a blood-carried molecule or cell, such as serum albumin (e.g., human serum albumin), IgG, erythrocytes, etc.

### IV. Pharmaceutical Compositions

Described herein are pharmaceutical compositions comprising an FcRn-antagonist and a pharmaceutically acceptable carrier or excipient. Examples of suitable pharmaceutical carriers are described in Remington's Pharmaceutical Sciences by E. W. Martin. Examples of excipients can include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The composition can also contain pH buffering reagents, and wetting or emulsifying agents.

The pharmaceutical composition can be formulated for parenteral administration (e.g., intravenous or intramuscular) by bolus injection. Formulations for injection can be presented in unit dosage form, e.g., in ampoules or in multidose containers with an added preservative. The compositions can take such forms as suspensions, solutions, or emulsions in oily or aqueous vehicles, and contain formulatory agents such as suspending, stabilizing and/or dispersing agents. Alternatively, the active ingredient can be in powder form for constitution with a suitable vehicle, e.g., pyrogen free water.

In thedescribed methods, FcRn-antagonists may be linked to chelators such as those described in U.S. Pat. No.5,326,856. The peptide-chelator complex may then be radiolabeled to provide an imaging agent for diagnosis or treatment of diseases or conditions involving the regulation of IgG levels.

### V. Exemplification

The present invention is further illustrated by the following examples, which should not be construed as further limiting. The invention shall be limited only be the scope of the appended claims.

### Example 1: Effect of Fc-Abdeg on serum IgG levels in cynomolgus monkeys

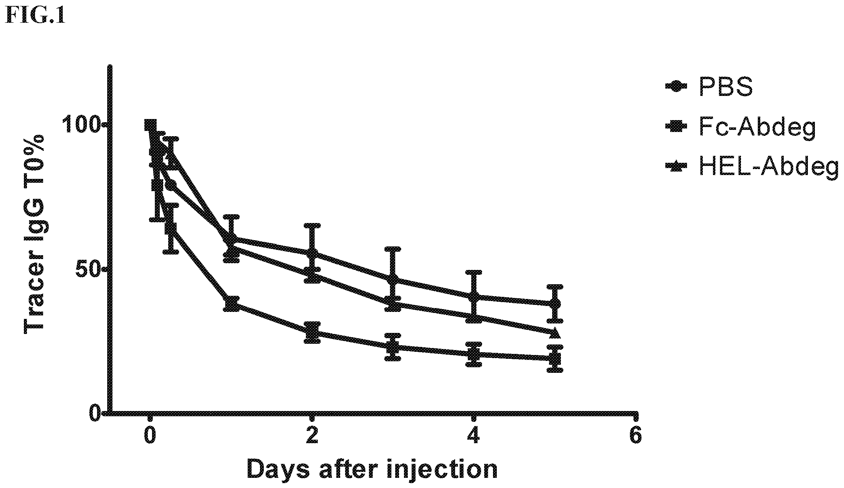

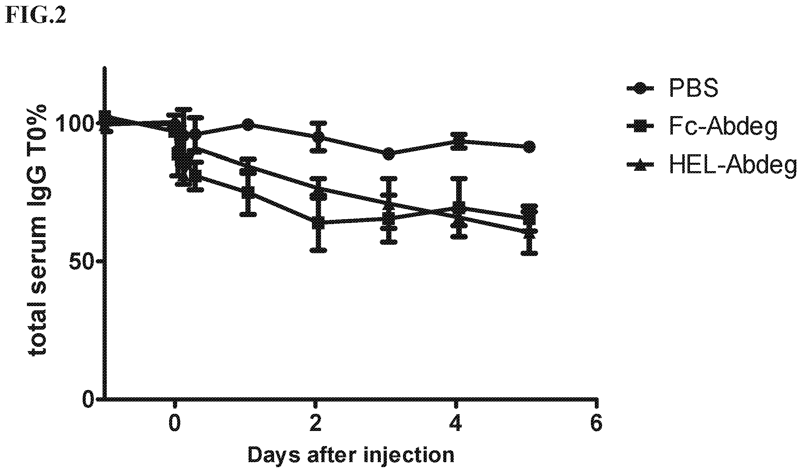

The effect of a human anti-lysozyme IgG (HEL-Abdeg) and a human IgG Fc region (Fc-Abdeg), comprising the amino acids Y, T, E, K, F, and Y at EU positions 252, 254, 256, 433, 434, and 436, respectively (Fc-Abdeg; SEQ ID NO:2), on serum IgG levels of a tracer antibody was determined in cynomolgus monkeys. Specifically, cynomolgus monkeys were administered 1mg/kg of an anti-murine CD70 hIgG1 tracer antibody (FR70-hIgG1; Oshima et al., Int Immunol 10(4): 517-26 (1998)) by i.v. bolus injection. Animals were infused 5 minutes later with either 7 mg/kg Fc-Abdeg, 20 mg/kg HEL-Abdeg, or PBS (2 monkeys per group). Infusion was performed within 1 hour and animals were administered a volume of 10 ml/kg. Blood samples (3x 150 µ1) were taken at 5 min prior to dosing ("pre-dose") and 5 min, 2h, 6h, 24h, 48h, 72h, 96h and 120h after completion of the infusion. Tracer levels were determined by performing a mCD70-binding ELISA and data were plotted relative to tracer levels at end of dosing (FIG.1). Total cynomolgus IgG levels were also determined (FIG.2). The results of these experiments show that Fc-Abdeg reduced tracer antibody more efficiently than equimolar amounts of HEL-Abdeg.

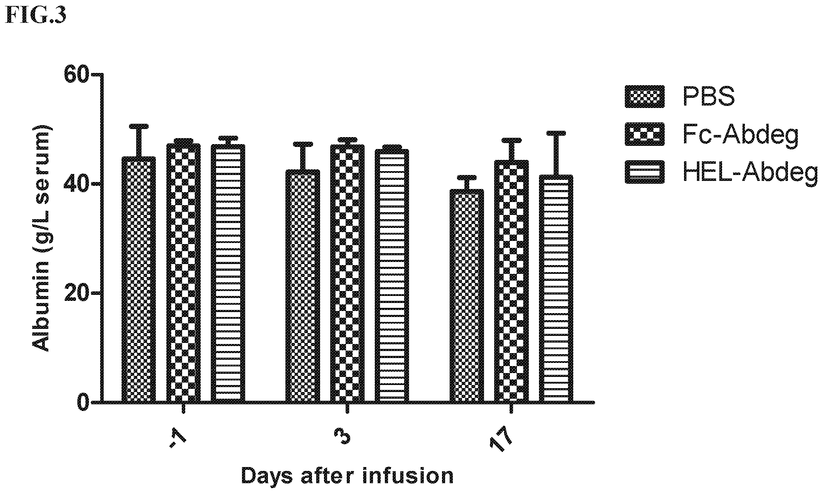

In addition to its key role in the IgG salvage pathway, FcRn is also involved in albumin homeostasis (Chaudhury et al., J Exp Med. 197(3):315-22 (2003). FcRn interacts with IgG-Fc and albumin at distinct sites and binding can happen concurrently (Andersen et al., Nat Commun. 3:610 (2012)). Conceptually, blockage of IgG recycling using Abdeg-modified molecules should not interfere with albumin-FcRn interaction. This hypothesis was confirmed in a mouse *in vivo* study, where the authors showed no influence of an Abdeg-equipped hIgG1 molecule on albumin levels (Patel et al., J Immunol 187(2): 1015-22 (2011)). In the experiment described above, albumin levels were also determined at day -3, day 3 and day 17 after the completion of the infusion. Analogous to the mouse study, no significant changes in albumin levels were observed after Fc-Abdeg or HEL-Abdeg treatment (see FIG.3).

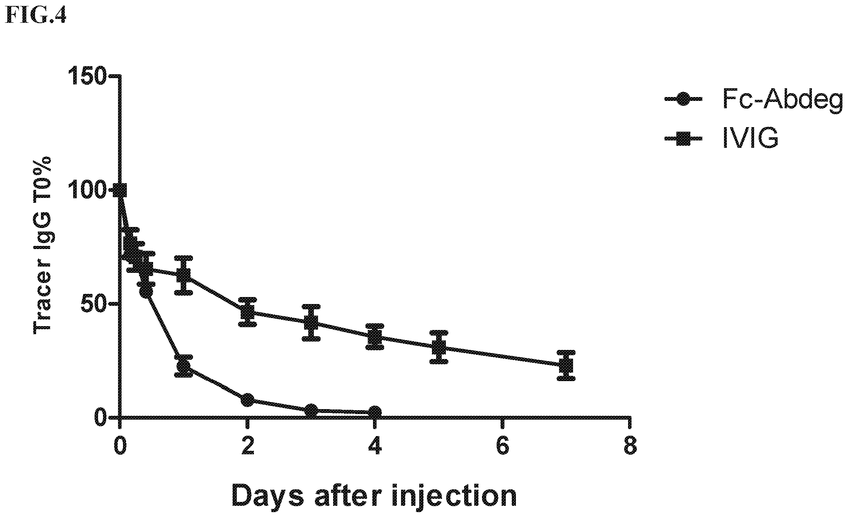

In a subsequent experiment, the antibody-depleting potency of Fc-Abdeg was compared to IVIG. Specifically, cynomolgus monkeys were administered with 1 mg/kg tracer antibody (FR70-hIgG1) 2 days prior to dosing with 70 mg/kg Fc-Abdeg or 2 g/kg IVIG (2 monkeys per group). Infusion of Fc-Abdeg and IVIG was performed within 4 hours and animals were administered a volume of 20 ml/kg. Blood (3x 150 µ1) samples were taken 5 min prior to dosing ("pre-dose"), and 5 min, 2h, 6h, 24h, 48h, 72h, 96h, 120h, and 168h after completion of the infusion. Tracer levels were determined by mCD70-binding ELISA and plotted relative to pre-dose levels (FIG.4). In comparison with IVIG treatment at clinical dose (2 g/kg), 70 mg/kg Fc-Abdeg showed significantly enhanced kinetics of tracer clearance and was also able to clear more efficiently (>95% tracer clearance in 4 days for Abdeg versus ~75% in 7 days for IVIG).

### Example 2: Effect of afucoslvation on Fc-Abdeg affinity for human CD16a and murine CD16-2

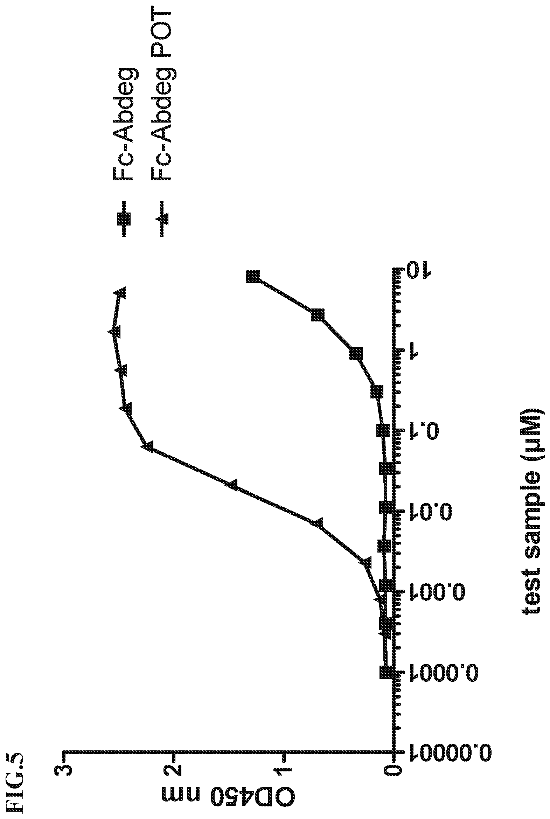

The binding affinity of Fc-Abdeg for hCD16a was determined and compared to the afucosylated form (Fc-Abdeg-POT). In the same experiment an Fc-Abdeg variant showing improved affinity for all FcγRs was included ("Fc-Abdeg-S239D/I332E). Specifically, a Maxisorp plate was coated with 100ng/well of Neutravidin Biotin-binding Protein (ThermoScientific, 31000) and incubated overnight at 4°C. The following day, the plate was blocked with PBS+1% casein for 2 hours at room temperature. Subsequently, 100µl/well of a 250ng/ml solution (dilution in PBS + 0.1% casein) of biotinylated hCD16a (Sino Biological Inc., 10389-H27H1-B) was added to the plate and incubated for 1 hour at room temperature prior to applying a concentration gradient of Fc-Abdeg or Fc-Abdeg-POT molecules (1 µM-0.005 nM) for a further hour. Binding to hCD16a was detected using an HRP-conjugated polyclonal goat anti-human Fc antibody (Jackson ImmunoResearch, 109-035-008) (incubation 1 hour at RT, dilution 1/50,000 in PBS+0.1% casein), followed by addition of 100µl room temperature-equilibrated TMB (SDT-reagents #s TMB). Plates were incubated for 10 minutes prior to addition of 100µl 0.5N H₂SO₄ and OD450nm measurement. EC50 values were determined using GraphPad Prism software. The results of these experiments, set forth in FIG.5, show that defucosylation of the Fc-Abdeg molecule results in a >30-fold increase in affinity for hCD16a (EC₅₀= 13nM for the Fc-Abdeg-POT vs. EC₅₀ >0.4µM for the fucosylated Fc-Abdeg). As expected, binding affinity of the Fc-Abdeg-S239D/I332E variant for hCD16a was increased compared to wild-type Fc-Abdeg (EC₅₀=6nM).

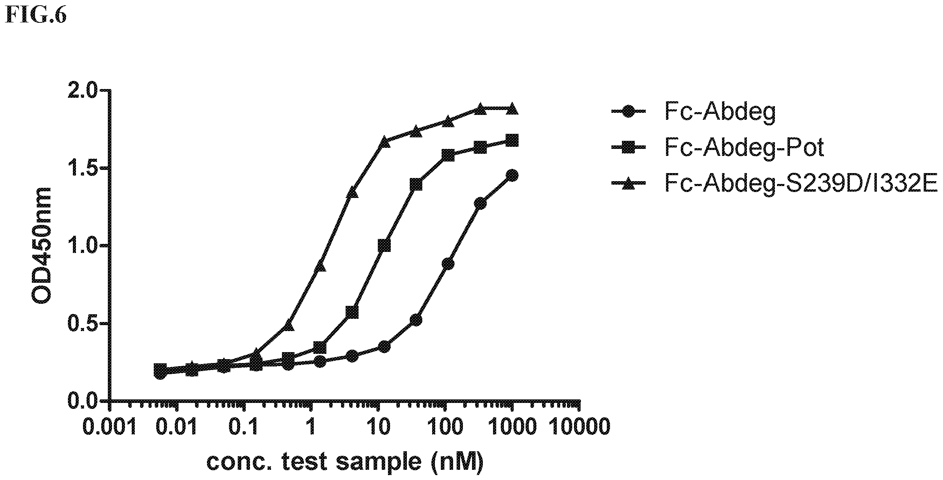

Using a similar experimental procedure as described above, the binding affinity for murine CD16-2 (Sino Biological Inc., 50036-M27H-B) was determined. The results of these experiments, set forth in FIG.6, again show an increased affinity of the afucosylated variant compared to the wild-type Fc-Abdeg (EC50=11nM vs. EC₅₀> 100nM). The fold increase in affinity for mCD16-2 of the Fc-Abdeg-POT variant over the wild-type Fc-Abdeg is lower compared to that observed for binding to human CD16a. This effect was not observed for the Fc-Abdeg-S239D/I332E variant (EC₅₀=2nM), which has a similar fold increase in affinity over wild type Fc-Abdeg for both the human and murine CD16 (EC₅₀=2nM).

Autoantibodies complexed with self antigens bind to activating FcγRs and thereby trigger the autoimmune diseases, which occur in part because of immunologically mediated inflammation against self tissue. The ability of Fc-Abdeg to antagonize the interaction of autoimmune antibodies and FcγRIII receptors on NK cells was evaluated in two ADCC-based assays.

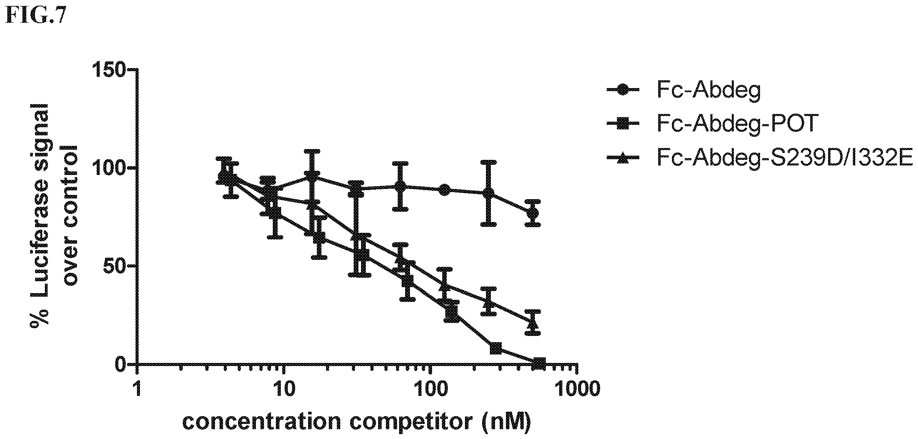

Initially, an ADCC reporter bioassay (Promega, G7016) was used to analyze the competitive hCD16a binding potency of Fc-Abdeg, Fc-Abdeg-POT and Fc-Abdeg-S239D/I332E. Specifically, 10,000 CD20-expressing Raji cells (target cells) were incubated with 60,000 Jurkat cells expressing hCD16a (effector cells) in presence of 100ng/ml anti-CD20 antibody and increasing concentration of competitor. Cells were incubated for 6 hours at 37°C prior to measuring the bioluminescence signal, which is a measure of ADCC-activity. The luciferase signal was plotted relative to the signal obtained by 100ng/ml anti-CD20 in the absence of competitor (see FIG.7). These experiments demonstrate that both Fc-Abdeg-POT and Fc-Abdeg-S239D/I332E efficiently block the anti-CD20-induced ADCC signal, whilst incubation with wild-type Fc-Abdeg does not lead to competitive binding for hCD16a expressed on Jurkat cells.

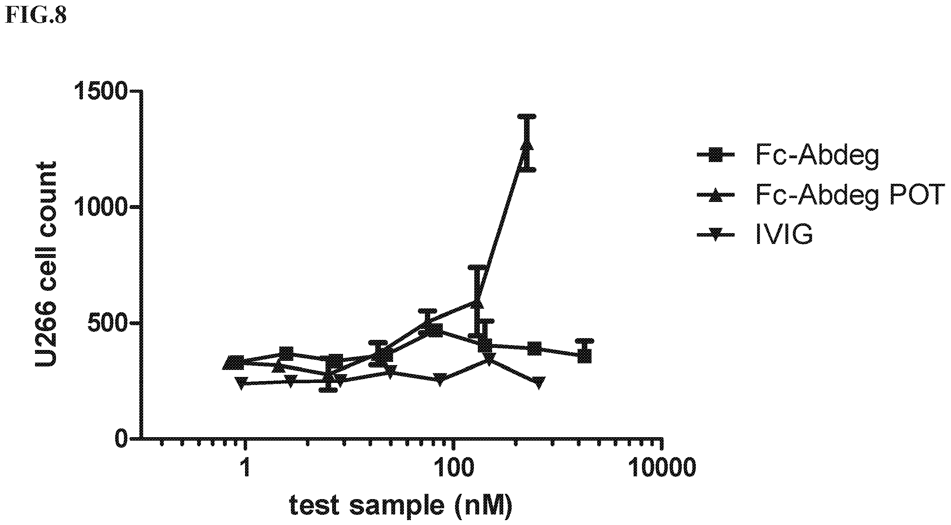

In a next ADCC assay, inhibition of the lytic activity of an anti-hCD70 antibody (27B3-hIgG1) by Fc-Abdeg and Fc-Abdeg-POT was tested as a measure of competitive hCD16 binding. Specifically, about 50,000 hCD70-expressing U266 cells were spiked into about 300,000 freshly purified PBMCs from a healthy donor in the presence of 50 ng/ml of the anti-hCD70 antibody and a concentration-gradient of Fc-Abdeg, Fc-Abdeg-POT and IVIG. The U266 cells were incubated for two days, and subsequent cell lysis was analyzed by FACS using a marker specific for the U266 cells (CD28). The results of these experiments, set forth in FIG.8, show that the anti-CD70 antibody efficiently lyses U266 cells and that this depletion could be attenuated in a dose-dependent fashion by addition of Fc-Abdeg-POT but not by wild-type Fc-Abdeg nor IVIG. These data demonstrate that Fc-Abdeg POT has enhanced competitive CD16a binding properties relative to wild-type Fc-Abdeg and IVIG.

### Example 3: Murine acute ITP model

The therapeutic potency of Fc-Abdeg, Fc-Abdeg-POT, Fc-Abdeg-S239D/I332E molecules was tested in a mouse model of acute immune thrombocytopenia. Specifically, C57BL/6 mice were treated with IVIG (20mg/animal), Fc-Abdeg (1 mg/animal), Fc-Abdeg-POT (1 mg/animal), Fc-Abdeg-S239D/I332E (1mg/animal) or saline via the intraperitoneal infusion (5 animals/group). Prior to treatment, a blood sample was withdrawn for a baseline measurement of platelet counts. One hour later, mice were treated with 5µg/animal of the anti-mouse platelet antibody MWReg30 (Nieswandt et al., Blood 94:684-93 (1999)). Platelet counts were monitored over 24 hours. Platelet counts were normalized relative to the initial counts for each mouse and platelets numbers were determined using flow cytometry via anti-CD61 staining. The results of these experiments, set forth in FIG.9, demonstrate that pretreatment with Fc-Abdeg reduces MWReg30-induced thrombocytopenia with a similar potency compared to a 7-fold higher molar dose of IVIG, and further, that blockade of FcγRs by Fc-Abdeg POT and Fc-Abdeg-S239D/I332E had a synergistic beneficial effect in this model, as seen by the improved platelet counts at the 180 and 1440 minutes time points.

### Example 4: Manufacturability Fc-Abdeg

Fc-Abdeg (comprising Fc domains having SEQ ID NO:2) was produced in CHO cells (Evitria, Switzerland) by transient transfection. Following transfection, high titers of Fc-Abdeg were detected in the supernatants (between 200 and 400 mg/ml). A similar favorable production profile was seen when Fc-Abdeg was expressed from an expression construct stably integrated into the CHO GS-XCEED cell line (Lonza, Great-Britain). On average, stable transfectants yielded 3g/L and several clones were identified which produced up to 6g/L Fc-Abdeg in a 10 L stirred tank bioreactor.

The manufacturability of the Fc-Abdeg was further investigated by analysis of aggregates and degradation products following protein A-purification of the aforementioned Fc-Abdeg production runs. Specifically, 137 µg of Fc-Abdeg was loaded on a Superdex 200 10/300 GL gelfiltration column (GE Healthcare) coupled to an ÄktaPurifier chromatography system. Results of this experiment, set forth in FIG.10, showed that only a very small percentage of Fc-Abdeg aggregates was observed (~0.5%), whilst no Fc-Abdeg degradation products were detected. Additionally, applying various stress conditions (freeze-thaw, rotational or temperature stress) to the protein A-purified Fc-Abdeg did not lead to any apparent change in physicochemical and functional properties. Taken together, these data demonstrate the excellent manufacturability of the Fc-Abdeg.

### Example 5: Dose-escalation study of Fc-Abdeg in cynomolgus monkeys

A dose-escalation study of Fc-Abdeg was performed in cynomolgus monkeys to determine the onset of the pharmacodynamics effect as well as the saturating dose. To this end, cynomolgus monkeys were administered 1 mg/kg of an anti-murine CD70 hIgG1 tracer antibody (FR70-hIgG1) by i.v. bolus injection. Animals were infused 48 hours later with various doses of FC-Abdeg (0.2 mg/kg, 2 mg/kg, 20mg/kg or 200mg/kg) or vehicle (PBS). Infusion was performed within 3 hours and animals were administered a volume of 36.36 ml/kg. Each test group consisted of 2 animals. Blood samples (3x150µl) were taken 5 minutes prior to dosing ("pre-dose") and 5 min, 2h, 6h, 24h, 48h, 72h, 5 days, 7 days, 10 days and 14 days after the end of the infusion. Both tracer IgG (see Figure 11) and endogenous IgG levels (see Figure 12) were determined by ELISA and plotted relative to pre-dose levels. Data from 70mg/kg dose were superposed from experiments set forth in Figure 4 herein. Dosing animals with 0.2 mg/kg FC-Abdeg did not significantly influence the rate of tracer clearance nor does it affect endogenous IgG levels. A clear pharmacodynamic effect was seen at 2mg/kg and this effect levels out at doses starting from 20mg/kg. A single administration of Fc-Abdeg reduces cynomolgus monkey IgGs by maximally 55% within 3-4 days.

FcRn binding is restricted to the gamma subtype of immunoglobulins, and is accountable for their much longer half-life compared to other immunoglobulin subtypes. Blocking the IgG recycling function of FcRn with Fc-Abdeg should therefore not interfere with endogenous non-IgG immunoglobulin levels, a feature which was demonstrated by measuring endogenous IgA and IgM levels in the serum samples of animals treated with 200mg/kg Fc-Abdeg (see Figures 13 and 14). Together with the observation set forth in Example 1 showing that Fc-Abdeg treatment does not influence albumin levels, these data demonstrate the specific pharmacodynamic effect of Fc-Abdeg.

Next, the pharmacokinetic profile of Fc-Abdeg was determined via ELISA. Fc-Abdeg has a short half-life (estimated half-life ~1.5 days) as has been calculated from its PK profile (see Figure 5). At saturating levels, non-FcRn binding Fc-Abdeg will be cleared efficiently due to its own mode of action. In addition, the molecular size of Fc-Abdeg is close to the cutoff for renal clearance (~60kDa).

### Example 6: Repetitive dosing study of Fc-Abdeg in cynomolgus monkeys

A single infusion of Fc-Abdeg at a dose of 20mg/kg reduces endogenous IgG levels by 55% in cynomolgus monkeys in approximately 3-4 days. In a subsequent experiment, it was assayed whether repetitive dosing of Fc-Abdeg would lower these levels even more. Two strategies were followed: one group of monkeys received an infusion of the test component every 24h during the first 4 days (days 1, 2, 3, 4), whilst the other group received the drug every four days (days 1, 5, 9, 13). For each individual administration, Fc-Abdeg was administered at a dose of 20mg/kg. Each test group consisted of 2 animals. Infusion procedure was identical to the one described in the Example 1. A clear pharmacodynamic difference between the two different groups is observed as of day 7 (see Figure 16). Dosing Fc-Abdeg every day for the first 4 days showed a similar reduction in IgG levels as a single infusion at the same dose (dashed line, data from Example 1 superposed as reference). Dosing Fc-Abdeg once every four days results in a more profound and longer reduction of these levels. A maximal reduction up to 25% of initial levels is observed in this treatment group.

The pharmacokinetic profile of Fc-Abdeg was determined (see Figure 7) and corresponds to the findings from the dose-escalation study set forth in Example 5.

### Example 7: Further dose-escalation study of Fc-Abdeg in cynomolgus monkeys

A follow-up dose-escalation study of Fc-Abdeg was performed in cynomolgus monkeys to further explore the onset and saturation of the pharmacodynamics effects. To this end, animals were infused with various doses of FC-Abdeg (10 mg/kg, 30 mg/kg, 50 mg/kg and 100mg/kg) or vehicle (PBS). Infusion was performed within two hours and each cohort consisted of four animals (two males and two females). Endogenous IgG levels were determined by ELISA and plotted relative to pre-dose levels (see Figure 18). An intermediate pharmacodynamic effect was observed at the 10mg/kg dose and this effect leveled out at doses starting from 30mg/kg. The rate of IgG depletion, onset of action and pharmacokinetic profile (see Figure 19, see Table 2) were similar to the findings set forth in Example 5.

**Table 2. Pharmacokinetic properties of Fc-Abdeg in cynomolgus monkeys**

| **Non-compartment analysis of Fc-Abdeg** | | | | | | | | | |
|---|---|---|---|---|---|---|---|---|---|
| **Dose [mg/kg]** | **Cmax^{#} [µg/mL]** | **tmax^{#} [h]** | **t_{1/2} [h]** | **tₗₐₛₜ^{#} [h]** | **CL [mL/h/kg]** | **AUC_{0-t last} [µg^{∗}h/mL]** | **AUC_{0-∞} [µg^{∗}h/mL]** | **AUC_{0-∞} /dose [µg^{∗}h^{∗}kg/** | **DPF** |
| **MALES** | | | | | | | | | |
| **10** | 179.1 | 2.0 | 22.8 | 672.0 | 1.95 | 5138 | 5138 | 514 | - |
| **30** | 512.3 | 2.0 | 24.7 | 672.0 | 1.99 | 15317 | 15317 | 511 | 0.99 |
| **50** | 850.7 | 2.0 | 26.0 | 672.0 | 1.93 | 26025 | 26025 | 521 | 1.01 |
| **100** | 1414.9 | 2.0 | 28.2 | 540.0 | 2.28 | 43865 | 43865 | 439 | 0.85 |

| **FEMALES** | | | | | | | | | |
|---|---|---|---|---|---|---|---|---|---|
| **10** | 188.3 | 2.0 | 21.3 | 672.0 | 1.80 | 5573 | 5573 | 557 | - |
| **30** | 447.6 | 2.0 | 34.5 | 672.0 | 2.13 | 14094 | 14094 | 470 | 0.84 |
| **50** | 978.9 | 2.0 | 27.5 | 456.0 | 1.78 | 28101 | 28104 | 562 | 1.01 |
| **100** | 1586.8 | 2.0 | 28.1 | 576.0 | 2.02 | 49553 | 49553 | 496 | 0.89 |

| | | | | | | | | | |
|---|---|---|---|---|---|---|---|---|---|
| #: Values obtained from serum analysis of Fc-Abdeg, all other values calculated by toxicokinetic analysis. -: not computable or no reasonable interpretation from the available data DPF: Dose proportion factor = [AUC_{0-t last} (x mg/kg)/AUC_{0-t last} (10 mg/kg)]/ [(x mg/kg)/ (10 mg/kg)] | | | | | | | | | |

### Example 8: Further repetitive dosing study of Fc-Abdeg in cynomolgus monkeys

A follow-up repetitive dosing study of Fc-Abdeg was performed in cynomolgus monkeys to further examine the pharmacodynamic effect of chronic Fc-Abdeg administration. To this end, animals were infused every 48h with various doses of FC-Abdeg (3 mg/kg, 30 mg/kg and 100mg/kg) or vehicle (PBS) for a total of 15 infusions. After the treatment period a recovery period of 30 days was included. Each cohort consisted of four animals, with the exception of the 100 mg/kg cohort (which consisted of three animals). Endogenous IgG levels were determined by ELISA and plotted relative to pre-dose levels (see Figure 20, average ± SEM). A clear drop in IgG levels was observed for all tested doses, and this pharmacodynamic effect persisted until the end of the treatment period. After the treatment period, IgG levels were restored to baseline within 10 days. The pharmacokinetic profile is shown in Figure 21.

### Example 9: Chronic dosing study of Fc-Abdeg in cynomolgus monkeys

Cynomolgus monkeys were infused with an i.v. loading dose of 20mg/kg FC-Abdeg and further received daily subcutaneous administration of Fc-Abdeg at 1, 3, or 5 mg/kg beginning 24 hours after the initial dose and continuing for for 12 days. Each test group consisted of two animals. IgG levels were determined by ELISA and plotted relative to pre-dose levels (see Figure 22). Data is also presented for a single cynomolgus monkey from the 3 mg/kg cohort in which the treatment persisted beyond 12 days. This monkey was infused with an i.v. loading dose of 20mg/kg FC-Abdeg, received daily subcutaneous administration of Fc-Abdeg at 3 mg/kg beginning 24 hours after the initial dose and continuing for 28 days, and was followed for a further treatment-free period of 32 days (see Figure 23). IgG levels were reduced up to 60% of original levels and remained consistently low throughout the treatment period. After the treatment period, IgG levels restored to baseline levels within two weeks.

## Claims

1. An isolated FcRn-antagonist consisting of a variant Fc region for use in a method of treating an autoimmune disease in a subject, the method comprising administering to the subject an effective amount of the isolated FcRn-antagonist consisting of the variant Fc region wherein the amino acid sequence of the Fc domains of the variant Fc region consists of the amino acid sequence set forth in SEQ ID NO:1, 2, or 3,
wherein the FcRn-antagonist is administered intravenously,
wherein the FcRn-antagonist is administered to the subject in a dose of between 2 and 200 mg/kg, and
wherein the FcRn-antagonist is administered to the subject at a frequency of once every 7 days.

2. The isolated FcRn-antagonist for use according to claim 1, wherein the amino acid sequence of the Fc domains of the variant Fc region consists of the amino acid sequence set forth in SEQ ID NO:1.

3. The isolated FcRn-antagonist for use according to claim 1, wherein the amino acid sequence of the Fc domains of the variant Fc region consists of the amino acid sequence set forth in SEQ ID NO:2.

4. The isolated FcRn-antagonist for use according to claim 1, wherein the amino acid sequence of the Fc domains of the variant Fc region consists of the amino acid sequence set forth in SEQ ID NO:3.

5. The isolated FcRn-antagonist for use according to any one of the preceding claims, wherein the FcRn-antagonist is linked to a half-life extender, optionally wherein the half-life extender is polyethylene glycol or human serum albumin.

6. The isolated FcRn-antagonist for use according to any one of the preceding claims, wherein the autoimmune disease is selected from the group consisting of allogenic islet graft rejection, alopecia areata, ankylosing spondylitis, antiphospholipid syndrome, autoimmune Addison's disease, Alzheimer's disease, antineutrophil cytoplasmic autoantibodies (ANCA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune disfunction syndrome, chronic inflammatory demyelinating polyneuropathy (CIDP), Churg-Strauss syndrome, cicatrical pemphigoid, CREST syndrome, cold agglutinin disease, Crohn's disease, dermatomyositis, dilated cardiomyopathy, discoid lupus, epidermolysis bullosa acquisita, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic membranous neuropathy, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen plantus, lichen sclerosus, lupus erthematosis, Meniere's disease, mixed connective tissue disease, mucous membrane pemphigoid, multiple sclerosis, type 1 diabetes mellitus, Multifocal motor neuropathy (MMN), myasthenia gravis, paraneoplastic bullous pemphigoid, pemphigoid gestationis, pemphigus vulgaris, pemphigus foliaceus, pernicious anemia, polyarteritis nodosa, polychrondritis, polyglandular syndromes, polymyalgia rheumatica, polymyositis and dermatomyositis, primary agammaglobinulinemia, primary biliary cirrhosis, psoriasis, psoriatic arthritis, relapsing polychondritis, Reynauld's phenomenon, Reiter's syndrome, rheumatoid arthritis, sarcoidosis, scleroderma, Sjorgen's syndrome, solid organ transplant rejection, stiff-man syndrome, systemic lupus erythematosus, takayasu arteritis, toxic epidermal necrolysis (TEN), Stevens Johnson syndrome (SJS), temporal arteristis/giant cell arteritis, thrombotic thrombocytopenia purpura, ulcerative colitis, uveitis, dermatitis herpetiformis vasculitis, anti-neutrophil cytoplasmic antibody-associated vasculitides, vitiligo, and Wegner's granulomatosis.

7. The isolated FcRn-antagonist for use according to any one of the preceding claims, wherein the disease is an autoimmune channelopathy, optionally wherein the channelopathy is selected from the group consisting of autoimmune limbic encephalitis, epilepsy, neuromyelitis optica, Lambert-Eaton myasthenic syndrome, myasthenia gravis, anti- N-Methyl-D-aspartate (NMDA) receptor encephalitis, anti-α-Amino-3-hydroxy-5-methyl-4-isoxazolepropionic acid (AMPA) receptor encephalitis, Morvan syndrome, neuromyotonia, pediatric autoimmune neuropychiatric disorders associated with streptococcal infection (PANDAS), and Glycine receptor antibody-associated disorder, or wherein the antibody-mediated disease or disorder is hyperglobulinemia.

8. The isolated FcRn-antagonist for use according to any one of the preceding claims, wherein the FcRn antagonist is administered to the subject simultaneously or sequentially with an additional therapeutic agent, optionally wherein the additional therapeutic agent is an anti-inflammatory agent or a leucocyte depleting agent.

9. The isolated FcRn-antagonist for use according to claim 7, wherein the leucocyte depleting agent is a B-cell depleting agent, optionally an antibody, optionally an antibody that specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86, optionally rituximab, daclizumab, basiliximab, muronomab-CD3, infliximab, adalimumab, omalizumab, efalizumab, natalizumab, tocilizumab, eculizumab, golimumab, canakinumab, ustekinumab, belimumab, or a combination thereof.

## Patentansprüche

1. Isolierter FcRn-Antagonist, bestehend aus einer varianten Fc-Region zur Verwendung in einem Verfahren zur Behandlung einer Autoimmunkrankheit in einem Individuum, wobei das Verfahren Verabreichen einer effektiven Menge des isolierten FcRn-Antagonisten, bestehend aus der varianten Fc-Region, an das Individuum umfasst, wobei die Aminosäuresequenz der Fc-Domänen der varianten Fc-Region aus der in SEQ ID NO: 1, 2, oder 3 dargestellten Aminosäuresequenz besteht,
wobei der FcRn-Antagonist intravenös verabreicht wird,
wobei der FcRn-Antagonist dem Individuum in einer Dosis zwischen 2 und 200 mg/kg verabreicht wird, und
wobei der FcRn-Antagonist dem Individuum mit einer Häufigkeit von einmal alle 7 Tage verabreicht wird.

2. Isolierter FcRn-Antagonist zur Verwendung nach Anspruch 1, wobei die Aminosäuresequenz der Fc-Domänen der varianten Fc-Region aus der in SEQ ID NO: 1 dargestellten Aminosäuresequenz besteht.

3. Isolierter FcRn-Antagonist zur Verwendung nach Anspruch 1, wobei die Aminosäuresequenz der Fc-Domänen der varianten Fc-Region aus der in SEQ ID NO: 2 dargestellten Aminosäuresequenz besteht.

4. Isolierter FcRn-Antagonist zur Verwendung nach Anspruch 1, wobei die Aminosäuresequenz der Fc-Domänen der varianten Fc-Region aus der in SEQ ID NO: 3 dargestellten Aminosäuresequenz besteht.

5. Isolierter FcRn-Antagonist zur Verwendung nach einem der vorhergehenden Ansprüche, wobei der FcRn-Antagonist mit einem Halbwertszeitverlängerer verknüpft ist, gegebenenfalls wobei der Halbwertszeitverlängerer Polyethylenglykol oder humanes Serumalbumin ist.

6. Isolierter FcRn-Antagonist zur Verwendung nach einem der vorhergehenden Ansprüche, wobei die Autoimmunkrankheit ausgewählt ist aus der Gruppe bestehend aus allogener Inseltransplantatabstoßung, Alopecia areata, ankylosierender Spondylitis, Antiphospholipid-Syndrom, autoimmuner Addison-Krankheit, Alzheimer-Krankheit, antineutrophilen zytoplasmatischen Autoantikörpern (ANCA), Autoimmunkrankheiten der Nebenniere, autoimmunhämolytische Anämie, Autoimmunhepatitis, Autoimmunmyokarditis, Autoimmunneutropenie, Autoimmunoophoritis und -orchitis, Autoimmun-Thrombozytopenie, Autoimmunurtikaria, Morbus Behcet, bullöses Pemphigoid, Kardiomyopathie, Castleman-Syndrom, Zöliakie-Sprue-Dermatitis, chronischem Müdigkeits-Immundysfunktionssyndrom, chronischer entzündlicher demyelinisierender Polyneuropathie (CIDP), Churg-Strauss-Syndrom, vernarbendem Pemphigoid, CREST-Syndrom, Kälteagglutininkrankheit, Morbus Crohn, Dermatomyositis, dilatativer Kardiomyopathie, diskoidem Lupus, Epidermolysis bullosa acquisita, essentieller gemischter Kryoglobulinämie, Faktor-VIII-Mangel, Fibromyalgie-Fibromyositis, Glomerulonephritis, Morbus Basedow, Guillain-Barre, Goodpasture-Syndrom, Graft-versus-Host-Krankheit (GVHD), Hashimoto-Thyreoiditis, Hämophilie A, idiopathischer membranöser Neuropathie, idiopathischer Lungenfibrose, idiopathischer Thrombozytopenie Purpura (ITP), IgA-Neuropathie, IgM-Polyneuropathien, immunvermittelter Thrombozytopenie, juveniler Arthritis, Kawasaki-Krankheit, Lichen Plantus, Lichen sclerosus, Lupus erthematosus, Morbus Meniere, Mischkollagenose, Schleimhautpemphigoid, Multipler Sklerose, Diabetes Mellitus Typ 1, multifokaler motorischer Neuropathie (MMN), Myasthenia gravis, paraneoplastischem bullösem Pemphigoid, Pemphigoid gestationis, Pemphigus vulgaris, Pemphigus foliaceus, perniziöser Anämie, Polyarteriitis nodosa, Polychrondritis, polyglandulären Syndromen, Polymyalgia rheumatica, Polymyostitis und Dermatomyostitis, primärer Agammaglobinulinemie, primärer biliärer Zirrhose, Psoriasis, Psoriasis-Arthritis, rezidivierender Polychondritis, Reynauld-Phänomen, Reiter-Syndrom, rheumatoider Arthritis, Sarkoidose, Sklerodermie, Sjörgen-Syndrom, Abstoßung von soliden Organtransplantaten, Stiff-Man-Syndrom, systemischem Lupus erythematodes, Takayasu-Arteriitis, toxischer epidermaler Nekrolyse (TEN), Stevens Johnson Syndrom (SJS), Arteriitis temporalis/Riesenzellarteriitis, thrombotischer Thrombozytopenie purpura, Colitis ulcerosa, Uveitis, Dermatitis herpetiformis vaskulitis, antineutrophilen zytoplasmatischen Antikörper-assoziierten Vaskulitiden, Vitiligo und Wegner-Granulomatose.

7. Isolierter FcRn-Antagonist zur Verwendung nach einem der vorhergehenden Ansprüche, wobei die Krankheit eine Autoimmunkanalopathie ist, gegebenenfalls, wobei die Kanalopathie ausgewählt ist aus der Gruppe bestehend aus autoimmuner limbischer Enzephalitis, Epilepsie, Neuromyelitis optica, Lambert-Eaton myasthenem Syndrom, Myasthenia gravis, Anti-N-Methyl-D-Aspartat (NMDA)-Rezeptor-Enzephalitis, Anti-α-Amino-3-hydroxy-5-methyl-4-isoxazolpropionsäure (AMPA)-Rezeptor-Enzephalitis, Morvan-Syndrom, Neuromyotonie, pädiatrischen neuropsychiatrischen Autoimmunkrankheiten im Zusammenhang mit einer Streptokokken-Infektion (PANDAS) und Glycin-Rezeptor-Antikörper-assoziierte Krankheit, oder wobei die Antikörpervermittelte Krankheit oder Störung Hyperglobulinämie ist.

8. Isolierter FcRn-Antagonist zur Verwendung nach einem der vorhergehenden Ansprüche, wobei der FcRn-Antagonist dem Individuum gleichzeitig oder sequentiell mit einem zusätzlichen Wirkstoff verabreicht wird, gegebenenfalls wobei der zusätzliche Wirkstoff ein antiinflammatorisches Mittel oder ein Leukozyten abbauendes Mittel ist.

9. Isolierter FcRn-Antagonist zur Verwendung nach Anspruch 7, wobei das Leukozyten abbauende Mittel ein B-Zellen abbauendes Mittel ist, gegebenenfalls ein Antikörper, gegebenenfalls ein Antikörper, der spezifisch an CD10, CD19, CD20, CD21, CD22, CD23, CD24, CD37, CD53, CD70, CD72, CD74, CD75, CD77, CD79a, CD79b, CD80, CD81, CD82, CD83, CD84, CD85 oder CD86 bindet, gegebenenfalls Rituximab, Daclizumab, Basiliximab, Muronomab-CD3, Infliximab, Adalimumab, Omalizumab, Efalizumab, Natalizumab, Tocilizumab, Eculizumab, Golimumab, Canakinumab, Ustekinumab, Belimumab, oder eine Kombination davon.

## Revendications

1. Antagoniste de FcRn isolé consistant en une région Fc variante pour une utilisation dans un procédé de traitement d'une maladie auto-immune chez un sujet, le procédé comprenant l'étape consistant à administrer au sujet une quantité efficace de l'antagoniste de FcRn isolé consistant en la région Fc variante où la séquence d'acides aminés des domaines Fc de la région Fc variante consiste en la séquence d'acides aminés représentée dans la SEQ ID NO : 1, 2 ou 3,
où l'antagoniste de FcRn est administré par voie intraveineuse,
où l'antagoniste de FcRn est administré au sujet à une dose comprise entre 2 et 200 mg/kg, et
où l'antagoniste de FcRn est administré au sujet à une fréquence d'une fois tous les 7 jours.

2. Antagoniste de FcRn isolé pour une utilisation selon la revendication 1, dans lequel la séquence d'acides aminés des domaines Fc de la région Fc variante consiste en la séquence d'acides aminés représentée dans la SEQ ID NO : 1.

3. Antagoniste de FcRn isolé pour une utilisation selon la revendication 1, dans lequel la séquence d'acides aminés des domaines Fc de la région Fc variante consiste en la séquence d'acides aminés représentée dans la SEQ ID NO : 2.

4. Antagoniste de FcRn isolé pour une utilisation selon la revendication 1, dans lequel la séquence d'acides aminés des domaines Fc de la région Fc variante consiste en la séquence d'acides aminés représentée dans la SEQ ID NO : 3.

5. Antagoniste de FcRn isolé pour une utilisation selon l'une quelconque des revendications précédentes, où l'antagoniste de FcRn est lié à un prolongateur de demi-vie, éventuellement où le prolongateur de demi-vie est du polyéthylèneglycol ou l'albumine sérique humaine.

6. Antagoniste de FcRn isolé pour une utilisation selon l'une quelconque des revendications précédentes, où la maladie auto-immune est choisie dans le groupe consistant en un rejet de greffe d'îlots allogéniques, l'alopécie en aires, la spondylarthrite ankylosante, le syndrome des antiphospholipides, la maladie auto-immune d'Addison, la maladie d'Alzheimer, les auto-anticorps anti-cytoplasme des neutrophiles (ANCA), les maladies auto-immunes de la glande surrénale, l'anémie hémolytique auto-immune, l'hépatite auto-immune, la myocardite auto-immune, la neutropénie auto-immune, l'orchite et l'oophorite auto-immunes, la thrombocytopénie auto-immune, l'urticaire auto-immune, la maladie de Behçet, la pemphigoïde bulleuse, la cardiomyopathie, le syndrome de Castleman, la maladie cœliaque-dermatite, le syndrome d'immunodysfonctionnement de fatigue chronique, la polyneuropathie démyélinisante inflammatoire chronique (PDIC), le syndrome de Churg-Strauss, la pemphigoïde cicatricielle, le syndrome de CREST, la maladie des agglutinines froides, la maladie de Crohn, la dermatomyosite, la cardiomyopathie dilatée, le lupus discoïde, l'épidermolyse bulleuse acquise, la cryoglobulinémie mixte essentielle, la déficience en facteur VIII, la fibromyalgie-fibromyosite, la glomérulonéphrite, la maladie de Graves, le syndrome de Guillain-Barré, le syndrome de Goodpasture, la réaction de greffe contre hôte (GVHD), la thyroïdite de Hashimoto, l'hémophilie A, la neuropathie membraneuse idiopathique, la fibrose pulmonaire idiopathique, la purpura thrombocytopénique idiopathique (PTI), la neuropathie à IgA, les polyneuropathies à IgM, la thrombocytopénie à médiation immunitaire, l'arthrite juvénile, la maladie de Kawasaki, le lichen plan, le lichen scléreux, le lupus érythémateux, la maladie de Ménière, la connectivite mixte, la pemphigoïde des muqueuses, la sclérose en plaques, le diabète sucré de type 1, la neuropathie motrice multifocale (MMN), la myasthénie gravis, la pemphigoïde bulleuse paranéoplasique, la pemphigoïde gestationis, le pemphigus vulgaire, le pemphigus foliacé, l'anémie pernicieuse, la polyartérite noueuse, la polychondrite, les syndromes polyglandulaires, la polymyalgie rhumatismale, la polymyosite et la dermatomyosite, l'agammaglobulinémie primaire, la cirrhose biliaire primitive, le psoriasis, l'arthrite psoriasique, la polychondrite récidivante, le phénomène de Raynaud, le syndrome de Reiter, la polyarthrite rhumatoïde, la sarcoïdose, la sclérodermie, le syndrome de Sjögren, le rejet de greffe d'organe solide, le syndrome de l'homme raide, le lupus érythémateux disséminé, l'artérite de Takayasu, la nécrolyse épidermique toxique (NET), le syndrome de Stevens-Johnson (SJS), l'artérite temporale/artérite à cellules géantes, la purpura thrombotique thrombocytopénique, la colite ulcéreuse, l'uvéite, la vascularite à dermatite herpétiforme, les vascularites associées à des anticorps anti-cytoplasme des neutrophiles, le vitiligo, et la granulomatose de Wegener.

7. Antagoniste de FcRn isolé pour une utilisation selon l'une quelconque des revendications précédentes, où la maladie est une canalopathie auto-immune, éventuellement où la canalopathie est choisie dans le groupe consistant en l'encéphalite limbique auto-immune, l'épilepsie, la neuromyélite optique, le syndrome myasthénique de Lambert-Eaton, la myasthénie gravis, l'encéphalite anti-récepteur de N-méthyl-D-aspartate (NMDA), l'encéphalite anti-récepteur de l'acide α-amino-3-hydroxy-5-méthyl-4-isoxazole-propionique (AMPA), le syndrome de Morvan, la neuromyotonie, des troubles neuropsychiatriques infantiles auto-immuns associés à des infections streptococciques (PANDAS), et un trouble associé à un anticorps anti-récepteur de la glycine, ou où la maladie ou le trouble à médiation par des anticorps est l'hyperglobulinémie.

8. Antagoniste de FcRn isolé pour une utilisation selon l'une quelconque des revendications précédentes, où l'antagoniste de FcRn est administré au sujet simultanément ou séquentiellement avec un agent thérapeutique supplémentaire, éventuellement où l'agent thérapeutique supplémentaire est un agent anti-inflammatoire ou un agent de déplétion leucocytaire.

9. Antagoniste de FcRn isolé pour une utilisation selon la revendication 7, où l'agent de déplétion leucocytaire est un agent de déplétion de cellules B, éventuellement un anticorps, éventuellement un anticorps qui se lie spécifiquement à CD10, CD19, CD20, CD21, CD22, CD23, CD24, CD37, CD53, CD70, CD72, CD74, CD75, CD77, CD79a, CD79b, CD80, CD81, CD82, CD83, CD84, CD85 ou CD86, éventuellement le rituximab, le daclizumab, le basiliximab, le muromonab-CD3, l'infliximab, l'adalimumab, l'omalizumab, l'efalizumab, le natalizumab, le tocilizumab, l'éculizumab, le golimumab, le canakinumab, l'ustékinumab, le belimumab, ou une combinaison de ceux-ci.
